# EUROPEAN PATENT SPECIFICATION

(11) **EP 1 631 234 B1**
(45) Date of publication and mention of the grant of the patent: **14.09.2011**
(21) Application number: 04752310.5
(22) Date of filing: 17.05.2004
(51) Int. Cl.: A01N 37/18, C07K 1/00, A61K 38/00

(54) **COMPOSITIONS AND METHODS FOR THE TREATMENT OF CNS INJURIES**
ZUSAMMENSETZUNGEN UND VERFAHREN ZUR BEHANDLUNG VON ZNS-SCHÄDEN
COMPOSITIONS ET METHODES DE TRAITEMENT DE BLESSURES DU SYSTEME NERVEUX CENTRAL

(30) Priority: 16.05.2003 US 471236 P
(43) Date of publication of application: 08.03.2006
(60) Divisional application: 11152626.5
(73) Proprietor: Acorda Therapeutics, Inc., Hawthorne, NY 10532 (US)
(72) Inventor: GRUSKIN, Elliott, A., Malvern, PA 19355 (US); CAGGIANO, Anthony, O., Larchmont, 10538 (US); ZIMBER, Michael, P., Mamroneck, 10543 (US); BLIGHT, Andrew, R., Mahopac, 10541 (US)
(74) Representative: Ouzman, Beverley Nicola Claire
(86) International application number: PCT/US2004/015253
(87) International publication number: WO 2004/103299

(56) References cited:
- WO-A-01/39795
- WO-A-99/46368
- WO-A-03/074080
- MODENA T ET AL: "HYALURONIDASE-INJECTABLE MICROPARTICLES INTENTED FOR THE TREATMENT OF EXTRAVASATION" JOURNAL OF MICROENCAPSULATION, TAYLOR AND FRANCIS, BASINGSTOKE, GB, vol. 15, no. 1, 1 January 1998 (1998-01-01), pages 85-92, XP000771652 ISSN: 0265-2048
- ZHUO ET AL.: 'Regeneration of Axons after nerve transection repair is enhanced by degradation of chondroitin sulfate proteoglycan' EXPERIMENTAL NEUROLOGY vol. 176, 2002, pages 221 - 228, XP003001915
- YASUDA ET AL.: 'Effect of hyaluronidase on experimental cerebral infarct size and mortality' LAB. INVEST. vol. 46, 1982, pages 400 - 404, XP009102217
- PILLWEIN ET AL.: 'Hyaluronidase additional to standard chemotherapy improves outcome for children with malignant tumors' CANCER LETTERS vol. 131, 1998, pages 101 - 108, XP009102216
- DOPPENBERG ET AL.: 'Clinical trials in traumatic brain injury' ANN. NY ACAD. SCI. 1998, pages 305 - 319, XP009102215

### SEQUENCE LISTING

<110> Acorda Therapeutics, Inc.
   Gruskin, Elliott A.
   Caggiano, Anthony O.
   Zimber, Michael P.
   Blight, Andrew R.
<120> COMPOSITIONS AND METHODS FOR THE TREATMENT OF CNS INJURIES
<130> 127304.02301
<140> not yet assigned
   <141> 2004-05-17
<150> 60/417,236
   <151> 2003-05-16
<160> 6
<170> PatentIn version 3.2
<210> 1
   <211> 2037
   <212> DNA
   <213> Flavobacterium heparinum
<400> 1
   cagcagaccg gtactgcaga actgattatg aagcgggtga tgctggacct taaaaagcct     60
   ttgcgcaata tggataaggt ggcggaaaag aacctgaata cgctgcagcc tgacggtagc    120
   tggaaggatg tgccttataa agatgatgcc atgaccaatt ggttgccaaa caaccacctg    180
   ctacaattgg aaactattat acaggcttat attgaaaaag atagtcacta ttatggcgac    240
   gataaagtgt ttgaccagat ttccaaagct tttaagtatt ggtatgacag cgacccgaaa    300
   agccgcaact ggtggcacaa tgaaattgcc actccgcagg cccttggtga aatgctgatc    360
   ctgatgcgtt acggtaaaaa gccgcttgat gaagcattgg tgcataaatt gaccgaaaga    420
   atgaagcggg gcgaaccgga gaagaaaacg ggggccaaca aaacagatat cgccctgcat    480
   tacttttatc gtgctttgtt aacgtctgat gaggctttgc tttccttcgc cgtaaaagaa    540
   ttgttttatc ccgtacagtt tgtacactat gaggaaggcc tgcaatacga ttattcctac    600
   ctgcagcacg gtccgcaatt acagatatcg agctacggtg ccgtatttat taccggggta    660
   ctgaaacttg ccaattacgt tagggatacc ccttatgctt taagtaccga gaaactggct    720
   atattttcaa agtattaccg cgacagttat ctgaaagcta tccgtggaag ttatatggat    780
   tttaacgtag aaggccgcgg agtaagccgg ccagacattc taaataaaaa ggcagaaaaa    840
   aagaggttgc tggtggcgaa gatgatcgat cttaagcata ctgaagaatg ggctgatgcg    900
   atagccagga cagatagcac agttgcggcc ggctataaga ttgagcccta tcaccatcag    960
   ttctggaatg gtgattatgt gcaacattta agacctgcct attcttttaa tgttcgtatg   1020
   gtgagtaagc ggacccgacg cagtgaatcc ggcaataaag aaaacctgct gggcaggtat   1080
   ttatctgatg gggctactaa catacaattg cgcggaccag aatactataa cattatgccg   1140
   gtatgggaat gggacaagat tcctggcata accagccgtg attatttaac cgacagacct   1200
   ttgacgaagc tttggggaga gcaggggagc aatgactttg caggaggggt gtctgatggt   1260
   gtatacgggg ccagtgccta cgcattggat tacgatagct tacaggcaaa gaaagcctgg   1320
   ttcttttttg acaaagagat tgtatgtctt ggtgccggta tcaacagcaa tgcccctgaa   1380
   aacattacca ctacccttaa ccagagctgg ttaaatggcc cggttataag tactgcaggt   1440
   aaaaccggcc ggggtaaaat aacaacgttt aaagcacagg gacagttctg gttgttgcac   1500
   gatgcgattg gttattactt tcctgaaggg gccaacctta gtctgagtac ccagtcgcaa   1560
   aaaggcaatt ggttccacat caacaattca cattcaaaag atgaagtttc tggtgatgta   1620
   tttaagcttt ggatcaacca tggtgccagg ccagaaaatg cgcagtatgc ttatatcgtt   1680
   ttgccgggaa taaacaagcc ggaagaaatt aaaaaatata atggaacggc accgaaagtc   1740
   cttgccaata ccaaccagct gcaggcagtt tatcatcagc agttagatat ggtacaggct   1800
   atcttctata cagctggaaa attaagcgta gcgggcgtag aaattgaaac agataagcca   1860
   tgtgcagtgc tgatcaagca catcaatggc aagcaggtaa tttgggctgc cgatccattg   1920
   caaaaagaaa agactgcagt gttgagcatc agggatttaa aaacaggaaa aacaaatcgg   1980
   gtaaaaattg attttccgca acaggaattt gcaggtgcaa cggttgaact gaaatag      2037
<210> 2
   <211> 1446
   <212> DNA
   <213> Flavobacterium heparinum
<400> 2
   caggttgttg cttcaaatga aactttatac caggttgtaa aggaggtaaa acccggtggt     60
   ctggtacaga ttgccgatgg gacttataaa gatgttcagc tgattgtcag caattcagga    120
   aaatctggtt tgcccatcac tattaaagcc ctgaacccgg gtaaggtttt ttttaccgga    180
   gatgctaaag tagagctgag gggcgagcac ctgatactgg aaggcatctg gtttaaagac    240
   gggaacagag ctattcaggc atggaaatca catggacccg gattggtggc tatatatggt    300
   agctataacc gcattaccgc atgtgtattt gattgttttg atgaagccaa ttctgcttac    360
   attactactt cgcttaccga agacggaaag gtacctcaac attgccgcat agaccattgc    420
   agttttaccg ataagatcac ttttgaccag gtaattaacc tgaacaatac agccagagct    480
   attaaagacg gttcggtggg aggaccgggg atgtaccatc gtgttgatca ctgttttttt    540
   tccaatccgc aaaaaccggg taatgccgga gggggaatca ggattggcta ttaccgtaat    600
   gatataggcc gttgtctggt agactctaac ctgtttatgc gtcaggattc ggaagcagag    660
   atcatcacca gcaaatcgca ggaaaatgtt tattatggta atacttacct gaattgccag    720
   ggcaccatga actttcgtca cggtgatcat caggtggcca ttaacaattt ttatataggc    780
   aatgaccagc gatttggata cgggggaatg tttgtttggg gaagcaggca tgtcatagcc    840
   tgtaattatt ttgagctgtc cgaaaccata aagtcgaggg ggaacgccgc attgtattta    900
   aaccccggtg ctatggcttc ggagcatgct cttgctttcg atatgttgat agccaacaac    960
   gctttcatca atgtaaatgg gtatgccatc cattttaatc cattggatga gcgcagaaaa   1020
   gaatattgtg cagccaatag gcttaagttc gaaaccccgc accagctaat gttaaaaggc   1080
   aatcttttct ttaaggataa accttatgtt tacccatttt ttaaagatga ttattttata   1140
   gcagggaaaa atagctggac tggtaatgta gccttaggtg tggaaaaggg aatccctgtt   1200
   aacatttcgg ccaataggtc tgcctataag ccggtaaaaa ttaaagatat ccagcccata   1260
   gaaggaatcg ctcttgatct caatgcgctg atcagcaaag gcattacagg aaagcccctt   1320
   agctgggatg aagtaaggcc ctactggtta aaagaaatgc ccgggacgta tgctttaacg   1380
   gccaggcttt ctgcagatag ggctgcaaag tttaaagccg taattaaaag aaataaagag   1440
   cactga                                                              1446
<210> 3
   <211> 2994
   <212> DNA
   <213> Proteus vulgaris
<220>
   <221> misc_feature
   <222> (1086)..(1086)
   <223> n is a, c, g, or t
<400> 3
   gccaccagca atcctgcatt tgatcctaaa aatctgatgc agtcagaaat ttaccatttt     60
   gcacaaaata acccattagc agacttctca tcagataaaa actcaatact aacgttatct    120
   gataaacgta gcattatggg aaaccaatct cttttatgga aatggaaagg tggtagtagc    180
   tttactttac ataaaaaact gattgtcccc accgataaag aagcatctaa agcatgggga    240
   cgctcatcca cccccgtttt ctcattttgg ctttacaatg aaaaaccgat tgatggttat    300
   cttactatcg atttcggaga aaaactcatt tcaaccagtg aggctcaggc aggctttaaa    360
   gtaaaattag atttcactgg ctggcgtact gtgggagtct ctttaaataa cgatcttgaa    420
   aatcgagaga tgaccttaaa tgcaaccaat acctcctctg atggtactca agacagcatt    480
   gggcgttctt taggtgctaa agtcgatagt attcgtttta aagcgccttc taatgtgagt    540
   cagggtgaaa tctatatcga ccgtattatg ttttctgtcg atgatgctcg ctaccaatgg    600
   tctgattatc aagtaaaaac tcgcttatca gaacctgaaa ttcaatttca caacgtaaag    660
   ccacaactac ctgtaacacc tgaaaattta gcggccattg atcttattcg ccaacgtcta    720
   attaatgaat ttgtcggagg tgaaaaagag acaaacctcg cattagaaga gaatatcagc    780
   aaattaaaaa gtgatttcga tgctcttaat actcacactt tagcaaatgg tggaacgcaa    840
   ggcagacatc tgatcactga taaacaaatc attatttatc aaccagagaa tcttaactct    900
   caagataaac aactatttga taattatgtt attttaggta attacacgac attaatgttt    960
   aatattagcc gtgcttatgt gctggaaaaa gatcccacac aaaaggcgca actaaagcag   1020
   atgtacttat taatgacaaa gcatttatta gatcaaggct ttgttaaagg gagtgcttta   1080
   gtgacnaccc atcactgggg atacagttct cgttggtggt atatttccac gttattaatg   1140
   tctgatgcac taaaagaagc gaacctacaa actcaagttt atgattcatt actgtggtat   1200
   tcacgtgagt ttaaaagtag ttttgatatg aaagtaagtg ctgatagctc tgatctagat   1260
   tatttcaata ccttatctcg ccaacattta gccttattac tactagagcc tgatgatcaa   1320
   aagcgtatca acttagttaa tactttcagc cattatatca ctggcgcatt aacgcaagtg   1380
   ccaccgggtg gtaaagatgg tttacgccct gatggtacag catggcgaca tgaaggcaac   1440
   tatccgggct actctttccc agcctttaaa aatgcctctc agcttattta tttattacgc   1500
   gatacaccat tttcagtggg tgaaagtggt tggaatagcc tgaaaaaagc gatggtttca   1560
   gcgtggatct acagtaatcc agaagttgga ttaccgcttg caggaagaca ccctcttaac   1620
   tcaccttcgt taaaatcagt cgctcaaggc tattactggc ttgccatgtc tgcaaaatca   1680
   tcgcctgata aaacacttgc atctatttat cttgcgatta gtgataaaac acaaaatgaa   1740
   tcaactgcta tttttggaga aactattaca ccagcgtctt tacctcaagg tttctatgcc   1800
   tttaatggcg gtgcttttgg tattcatcgt tggcaagata aaatggtgac actgaaagct   1860
   tataacacca atgtttggtc atctgaaatt tataacaaag ataaccgtta tggccgttac   1920
   caaagtcatg gtgtcgctca aatagtgagt aatggctcgc agctttcaca gggctatcag   1980
   caagaaggtt gggattggaa tagaatgcca ggggcaacca ctatccacct tcctcttaaa   2040
   gacttagaca gtcctaaacc tcatacctta atgcaacgtg gagagcgtgg atttagcgga   2100
   acatcatccc ttgaaggtca atatggcatg atggcattcg atcttattta tcccgccaat   2160
   cttgagcgtt ttgatcctaa tttcactgcg aaaaagagtg tattagccgc tgataatcac   2220
   ttaattttta ttggtagcaa tataaatagt agtgataaaa ataaaaatgt tgaaacgacc   2280
   ttattccaac atgccattac tccaacatta aatacccttt ggattaatgg acaaaagata   2340
   gaaaacatgc cttatcaaac aacacttcaa caaggtgatt ggttaattga tagcaatggc   2400
   aatggttact taattactca agcagaaaaa gtaaatgtaa gtcgccaaca tcaggtttca   2460
   gcggaaaata aaaatcgcca accgacagaa ggaaacttta gctcggcatg gatcgatcac   2520
   agcactcgcc ccaaagatgc cagttatgag tatatggtct ttttagatgc gacacctgaa   2580
   aaaatgggag agatggcaca aaaattccgt gaaaataatg ggttatatca ggttcttcgt   2640
   aaggataaag acgttcatat tattctcgat aaactcagca atgtaacggg atatgccttt   2700
   tatcagccag catcaattga agacaaatgg atcaaaaagg ttaataaacc tgcaattgtg   2760
   atgactcatc gacaaaaaga cactcttatt gtcagtgcag ttacacctga tttaaatatg   2820
   actcgccaaa aagcagcaac tcctgtcacc atcaatgtca cgattaatgg caaatggcaa   2880
   tctgctgata aaaatagtga agtgaaatat caggtttctg gtgataacac tgaactgacg   2940
   tttacgagtt actttggtat tccacaagaa atcaaactct cgccactccc ttga         2994
<210> 4
   <211> 997
   <212> PRT
   <213> Proteus Vulgaris
<400> 4
   Ala Thr Ser Asn Pro Ala Phe Asp Pro Lys Asn Leu Met Gln Ser Glu
   1               5                   10                  15
   Ile Tyr His Phe Ala Gln Asn Asn Pro Leu Ala Asp Phe Ser Ser Asp
               20                  25                  30
   Lys Asn Ser Ile Leu Thr Leu Ser Asp Lys Arg Ser Ile Met Gly Asn
           35                  40                  45
   Gln Ser Leu Leu Trp Lys Trp Lys Gly Gly Ser Ser Phe Thr Leu His
       50                  55                  60
   Lys Lys Leu Ile Val Pro Thr Asp Lys Glu Ala Ser Lys Ala Trp Gly
   65                  70                  75                  80
   Arg Ser Ser Thr Pro Val Phe Ser Phe Trp Leu Tyr Asn Glu Lys Pro
                   85                  90                  95
   Ile Asp Gly Tyr Leu Thr Ile Asp Phe Gly Glu Lys Leu Ile Ser Thr
               100                 105                 110
   Ser Glu Ala Gln Ala Gly Phe Lys Val Lys Leu Asp Phe Thr Gly Trp
           115                 120                 125
   Arg Thr Val Gly Val Ser Leu Asn Asn Asp Leu Glu Asn Arg Glu Met
       130                 135                 140
   Thr Leu Asn Ala Thr Asn Thr Ser Ser Asp Gly Thr Gln Asp Ser Ile
   145                 150                 155                 160
   Gly Arg Ser Leu Gly Ala Lys Val Asp Ser Ile Arg Phe Lys Ala Pro
                   165                 170                 175
   Ser Asn Val Ser Gln Gly Glu Ile Tyr Ile Asp Arg Ile Met Phe Ser
               180                 185                 190
   Val Asp Asp Ala Arg Tyr Gln Trp Ser Asp Tyr Gln Val Lys Thr Arg
           195                 200                 205
   Leu Ser Glu Pro Glu Ile Gln Phe His Asn Val Lys Pro Gln Leu Pro
       210                 215                 220
   Val Thr Pro Glu Asn Leu Ala Ala Ile Asp Leu Ile Arg Gln Arg Leu
   225                 230                 235                 240
   Ile Asn Glu Phe Val Gly Gly Glu Lys Glu Thr Asn Leu Ala Leu Glu
                   245                 250                 255
   Glu Asn Ile Ser Lys Leu Lys Ser Asp Phe Asp Ala Leu Asn Thr His
               260                 265                 270
   Thr Leu Ala Asn Gly Gly Thr Gln Gly Arg His Leu Ile Thr Asp Lys
           275                 280                 285
   Gln Ile Ile Ile Tyr Gln Pro Glu Asn Leu Asn Ser Gln Asp Lys Gln
       290                 295                 300
   Leu Phe Asp Asn Tyr Val Ile Leu Gly Asn Tyr Thr Thr Leu Met Phe
   305                 310                 315                 320
   Asn Ile Ser Arg Ala Tyr Val Leu Glu Lys Asp Pro Thr Gln Lys Ala
                   325                 330                 335
   Gln Leu Lys Gln Met Tyr Leu Leu Met Thr Lys His Leu Leu Asp Gln
               340                 345                 350
   Gly Phe Val Lys Gly Ser Ala Leu Val Thr Thr His His Trp Gly Tyr
           355                 360                 365
   Ser Ser Arg Trp Trp Tyr Ile Ser Thr Leu Leu Met Ser Asp Ala Leu
       370                 375                 380
   Lys Glu Ala Asn Leu Gln Thr Gln Val Tyr Asp Ser Leu Leu Trp Tyr
   385                 390                 395                 400
   Ser Arg Glu Phe Lys Ser Ser Phe Asp Met Lys Val Ser Ala Asp Ser
                   405                 410                 415
   Ser Asp Leu Asp Tyr Phe Asn Thr Leu Ser Arg Gln His Leu Ala Leu
               420                 425                 430
   Leu Leu Leu Glu Pro Asp Asp Gln Lys Arg Ile Asn Leu Val Asn Thr
           435                 440                 445
   Phe Ser His Tyr Ile Thr Gly Ala Leu Thr Gln Val Pro Pro Gly Gly
       450                 455                 460
   Lys Asp Gly Leu Arg Pro Asp Gly Thr Ala Trp Arg His Glu Gly Asn
   465                 470                 475                 480
   Tyr Pro Gly Tyr Ser Phe Pro Ala Phe Lys Asn Ala Ser Gln Leu Ile
                   485                 490                 495
   Tyr Leu Leu Arg Asp Thr Pro Phe Ser Val Gly Glu Ser Gly Trp Asn
               500                 505                 510
   Ser Leu Lys Lys Ala Met Val Ser Ala Trp Ile Tyr Ser Asn Pro Glu
           515                 520                 525
   Val Gly Leu Pro Leu Ala Gly Arg His Pro Leu Asn Ser Pro Ser Leu
       530                 535                 540
   Lys Ser Val Ala Gln Gly Tyr Tyr Trp Leu Ala Met Ser Ala Lys Ser
   545 550 555 560
   Ser Pro Asp Lys Thr Leu Ala Ser Ile Tyr Leu Ala Ile Ser Asp Lys
   565 570 575
   Thr Gln Asn Glu Ser Thr Ala Ile Phe Gly Glu Thr Ile Thr Pro Ala
   580 585 590
   Ser Leu Pro Gln Gly Phe Tyr Ala Phe Asn Gly Gly Ala Phe Gly Ile
   595 600 605
   His Arg Trp Gln Asp Lys Met Val Thr Leu Lys Ala Tyr Asn Thr Asn
   610 615 620
   Val Trp Ser Ser Glu Ile Tyr Asn Lys Asp Asn Arg Tyr Gly Arg Tyr
   625 630 635 640
   Gln Ser His Gly Val Ala Gln Ile Val Ser Asn Gly Ser Gln Leu Ser
   645 650 655
   Gln Gly Tyr Gln Gln Glu Gly Trp Asp Trp Asn Arg Met Pro Gly Ala
   660 665 670
   Thr Thr Ile His Leu Pro Leu Lys Asp Leu Asp Ser Pro Lys Pro His
   675 680 685
   Thr Leu Met Gln Arg Gly Glu Arg Gly Phe Ser Gly Thr Ser Ser Leu
   690 695 700
   Glu Gly Gln Tyr Gly Met Met Ala Phe Asp Leu Ile Tyr Pro Ala Asn
   705 710 715 720
   Leu Glu Arg Phe Asp Pro Asn Phe Thr Ala Lys Lys Ser Val Leu Ala
   725 730 735
   Ala Asp Asn His Leu Ile Phe Ile Gly Ser Asn Ile Asn Ser Ser Asp
   740 745 750
   Lys Asn Lys Asn Val Glu Thr Thr Leu Phe Gln His Ala Ile Thr Pro
   755 760 765
   Thr Leu Asn Thr Leu Trp Ile Asn Gly Gln Lys Ile Glu Asn Met Pro
   770 775 780
   Tyr Gln Thr Thr Leu Gln Gln Gly Asp Trp Leu Ile Asp Ser Asn Gly
   785 790 795 800
   Asn Gly Tyr Leu Ile Thr Gln Ala Glu Lys Val Asn Val Ser Arg Gln
   805 810 815
   His Gln Val Ser Ala Glu Asn Lys Asn Arg Gln Pro Thr Glu Gly Asn
   820 825 830
   Phe Ser Ser Ala Trp Ile Asp His Ser Thr Arg Pro Lys Asp Ala Ser
   835 840 845
   Tyr Glu Tyr Met Val Phe Leu Asp Ala Thr Pro Glu Lys Met Gly Glu
   850 855 860
   Met Ala Gln Lys Phe Arg Glu Asn Asn Gly Leu Tyr Gln Val Leu Arg
   865 870 875 880
   Lys Asp Lys Asp Val His Ile Ile Leu Asp Lys Leu Ser Asn Val Thr
   885 890 895
   Gly Tyr Ala Phe Tyr Gln Pro Ala Ser Ile Glu Asp Lys Trp Ile Lys
   900 905 910
   Lys Val Asn Lys Pro Ala Ile Val Met Thr His Arg Gln Lys Asp Thr
   915 920 925
   Leu Ile Val Ser Ala Val Thr Pro Asp Leu Asn Met Thr Arg Gln Lys
   930 935 940
   Ala Ala Thr Pro Val Thr Ile Asn Val Thr Ile Asn Gly Lys Trp Gln
   945 950 955 960
   Ser Ala Asp Lys Asn Ser Glu Val Lys Tyr Gln Val Ser Gly Asp Asn
   965 970 975
   Thr Glu Leu Thr Phe Thr Ser Tyr Phe Gly Ile Pro Gln Glu Ile Lys
   980 985 990
   Leu Ser Pro Leu Pro
   995
<210> 5
   <211> 2973
   <212> DNA
   <213> Proteus vulgaris
<400> 5
   ttacccactc tgtctcatga agctttcggc gatatttatc tttttgaagg cgaattaccc 60
   aatatcctta ccacttcaaa taataatcaa ttatcgctaa gcaaacagca tgctaaagat 120
   ggtgaacaat cactcaaatg gcaatatcaa ccacaagcaa cattaacact aaataatatt 180
   gttaattacc aagatgataa aaatacagcc acaccactca cttttatgat gtggatttat 240
   aatgaaaaac ctcaatcttc cccattaacg ttagcattta aacaaaataa taaaattgca 300
   ctaagtttta atgctgaact taattttacg gggtggcgag gtattgctgt tccttttcgt 360
   gatatgcaag gctctgcgac aggtcaactt gatcaattag tgatcaccgc tccaaaccaa 420
   gccggaacac tcttttttga tcaaatcatc atgagtgtac cgttagacaa tcgttgggca 480
   gtacctgact atcaaacacc ttacgtaaat aacgcagtaa acacgatggt tagtaaaaac 540
   tggagtgcat tattgatgta cgatcagatg tttcaagccc attaccctac tttaaacttc 600
   gatactgaat ttcgcgatga ccaaacagaa atggcttcga tttatcagcg ctttgaatat 660
   tatcaaggaa ttcgtagtga taaaaaaatt actccagata tgctagataa acatttagcg 720
   ttatgggaaa aattggggtt aacacaacac gctgatggct caatcacagg aaaagccctt 780
   gatcacccta accggcaaca ttttatgaaa gtcgaaggtg tatttagtga ggggactcaa 840
   aaagcattac ttgatgccaa tatgctaaga gatgtgggca aaacgcttct tcaaactgct 900
   atttacttgc gtagcgattc attatcagca actggtagaa aaaaattaga agagcgctat 960
   ttattaggta ctcgttatgt ccttgaacaa ggttttacac gaggaagtgg ttatcaaatt 1020
   attactcatg ttggttacca aaccagagaa ctttttgatg catggtttat tggccgtcat 1080
   gttcttgcaa aaaataacct tttagccccc actcaacaag ctatgatgtg gtacaacgcc 1140
   acaggacgta tttttgaaaa agataatgaa attgttgatg caaatgtcga tattctcaat 1200
   actcaattgc aatggatgat aaaaagctta ttgatgctac cggattatca acaacgtcaa 1260
   caagccttag cgcaactgca aagttggcta aataaaacca ttctaagctc aaaaggtgtt 1320
   gctggcggtt tcaaatctga tggttctatt tttcaccatt cacaacatta ccccgcttat 1380
   gctaaagatg catttggtgg tttagcaccc agtgtttatg cattaagtga ttcacctttt 1440
   cgcttatcta cttcagcaca tgagcattta aaagatgttt tgttaaaaat gcggatctac 1500
   accaaagaga cacaaattcc tgtggtatta agtggtcgtc atccaactgg gttgcataaa 1560
   atagggatcg cgccatttaa atggatggca ttagcaggaa ccccagatgg caaacaaaag 1620
   ttagatacca cattatccgc cgcttatgca aacttagaca acaaaacgca ttttgaaggc 1680
   attaacgctg aaagtgagcc agtcggcgca tgggcaatga attatgcatc aatggcaata 1740
   caacgaagag catcgaccca atcaccacaa caaagctggc tcgccatagc gcgcggtttt 1800
   agccgttatc ttgttggtaa tgaaagctat gaaaataaca accgttatgg tcgttattta 1860
   caatatggac aattggaaat tattccagct gatttaactc aatcagggtt tagccatgct 1920
   ggatgggatt ggaatagata tccaggtaca acaactattc atcttcccta taacgaactt 1980
   gaagcaaaac ttaatcaatt acctgctgca ggtattgaag aaatgttgct ttcaacagaa 2040
   agttactctg gtgcaaatac ccttaataat aacagtatgt ttgccatgaa attacacggt 2100
   cacagtaaat atcaacaaca aagcttaagg gcaaataaat cctatttctt atttgataat 2160
   agagttattg ctttaggctc aggtattgaa aatgatgata aacaacatac gaccgaaaca 2220
   acactattcc agtttgccgt ccctaaatta cagtcagtga tcattaatgg caaaaaggta 2280
   aatcaattag atactcaatt aactttaaat aatgcagata cattaattga tcctgccggc 2340
   aatttatata agctcactaa aggacaaact gtaaaattta gttatcaaaa acaacattca 2400
   cttgatgata gaaattcaaa accaacagaa caattatttg caacagctgt tatttctcat 2460
   ggtaaggcac cgagtaatga aaattatgaa tatgcaatag ctatcgaagc acaaaataat 2520
   aaagctccca aatacacagt attacaacat aatgatcagc tccatgcggt aaaagataaa 2580
   ataacccaag aagagggata tggttttttt gaagccacta agttaaaatc agcggatgca 2640
   acattattat ccagtgatgc gccggttatg gtcatggcta aaatacaaaa tcagcaatta 2700
   acattaagta ttgttaatcc tgatttaaat ttatatcaag gtagagaaaa agatcaattt 2760
   gatgataaag gtaatcaaat cgaagttagt gtttattctc gtcattggct tacagcagaa 2820
   tcgcaatcaa caaatagtac tattaccgta aaaggaatat ggaaattaac gacacctcaa 2880
   cccggtgtta ttattaagca ccacaataac aacactctta ttacgacaac aaccatacag 2940
   gcaacaccta ctgttattaa tttagttaag taa 2973
<210> 6
   <211> 990
   <212> PRT
   <213> Proteus vulgaris
<400> 6
   Leu Pro Thr Leu Ser His Glu Ala Phe Gly Asp Ile Tyr Leu Phe Glu
   1               5                   10                  15
   Gly Glu Leu Pro Asn Ile Leu Thr Thr Ser Asn Asn Asn Gln Leu Ser
               20                  25                  30
   Leu Ser Lys Gln His Ala Lys Asp Gly Glu Gln Ser Leu Lys Trp Gln
           35                  40                  45
   Tyr Gln Pro Gln Ala Thr Leu Thr Leu Asn Asn Ile Val Asn Tyr Gln
       50                  55                  60
   Asp Asp Lys Asn Thr Ala Thr Pro Leu Thr Phe Met Met Trp Ile Tyr
   65                  70                  75                  80
   Asn Glu Lys Pro Gln Ser Ser Pro Leu Thr Leu Ala Phe Lys Gln Asn
                   85                  90                  95
   Asn Lys Ile Ala Leu Ser Phe Asn Ala Glu Leu Asn Phe Thr Gly Trp
               100                 105                 110
   Arg Gly Ile Ala Val Pro Phe Arg Asp Met Gln Gly Ser Ala Thr Gly
           115                 120                 125
   Gln Leu Asp Gln Leu Val Ile Thr Ala Pro Asn Gln Ala Gly Thr Leu
       130                 135                 140
   Phe Phe Asp Gln Ile Ile Met Ser Val Pro Leu Asp Asn Arg Trp Ala
   145                 150                 155                 160
   Val Pro Asp Tyr Gln Thr Pro Tyr Val Asn Asn Ala Val Asn Thr Met
   165 170 175
   Val Ser Lys Asn Trp Ser Ala Leu Leu Met Tyr Asp Gln Met Phe Gln
   180 185 190
   Ala His Tyr Pro Thr Leu Asn Phe Asp Thr Glu Phe Arg Asp Asp Gln
   195 200 205
   Thr Glu Met Ala Ser Ile Tyr Gln Arg Phe Glu Tyr Tyr Gln Gly Ile
   210 215 220
   Arg Ser Asp Lys Lys Ile Thr Pro Asp Met Leu Asp Lys His Leu Ala
   225 230 235 240
   Leu Trp Glu Lys Leu Gly Leu Thr Gln His Ala Asp Gly Ser Ile Thr
   245 250 255
   Gly Lys Ala Leu Asp His Pro Asn Arg Gln His Phe Met Lys Val Glu
   260 265 270
   Gly Val Phe Ser Glu Gly Thr Gln Lys Ala Leu Leu Asp Ala Asn Met
   275 280 285
   Leu Arg Asp Val Gly Lys Thr Leu Leu Gln Thr Ala Ile Tyr Leu Arg
   290 295 300
   Ser Asp Ser Leu Ser Ala Thr Gly Arg Lys Lys Leu Glu Glu Arg Tyr
   305 310 315 320
   Leu Leu Gly Thr Arg Tyr Val Leu Glu Gln Gly Phe Thr Arg Gly Ser
   325 330 335
   Gly Tyr Gln Ile Ile Thr His Val Gly Tyr Gln Thr Arg Glu Leu Phe
   340 345 350
   Asp Ala Trp Phe Ile Gly Arg His Val Leu Ala Lys Asn Asn Leu Leu
   355 360 365
   Ala Pro Thr Gln Gln Ala Met Met Trp Tyr Asn Ala Thr Gly Arg Ile
   370 375 380
   Phe Glu Lys Asp Asn Glu Ile Val Asp Ala Asn Val Asp Ile Leu Asn
   385 390 395 400
   Thr Gln Leu Gln Trp Met Ile Lys Ser Leu Leu Met Leu Pro Asp Tyr
   405 410 415
   Gln Gln Arg Gln Gln Ala Leu Ala Gln Leu Gln Ser Trp Leu Asn Lys
   420 425 430
   Thr Ile Leu Ser Ser Lys Gly Val Ala Gly Gly Phe Lys Ser Asp Gly
           435                 440                 445
   Ser Ile Phe His His Ser Gln His Tyr Pro Ala Tyr Ala Lys Asp Ala
       450                 455                 460
   Phe Gly Gly Leu Ala Pro Ser Val Tyr Ala Leu Ser Asp Ser Pro Phe
   465                 470                 475                 480
   Arg Leu Ser Thr Ser Ala His Glu His Leu Lys Asp Val Leu Leu Lys
                   485                 490                 495
   Met Arg Ile Tyr Thr Lys Glu Thr Gln Ile Pro Val Val Leu Ser Gly
               500                 505                 510
   Arg His Pro Thr Gly Leu His Lys Ile Gly Ile Ala Pro Phe Lys Trp
           515                 520                 525
   Met Ala Leu Ala Gly Thr Pro Asp Gly Lys Gln Lys Leu Asp Thr Thr
       530                 535                 540
   Leu Ser Ala Ala Tyr Ala Asn Leu Asp Asn Lys Thr His Phe Glu Gly
   545                 550                 555                 560
   Ile Asn Ala Glu Ser Glu Pro Val Gly Ala Trp Ala Met Asn Tyr Ala
                   565                 570                 575
   Ser Met Ala Ile Gln Arg Arg Ala Ser Thr Gln Ser Pro Gln Gln Ser
               580                 585                 590
   Trp Leu Ala Ile Ala Arg Gly Phe Ser Arg Tyr Leu Val Gly Asn Glu
           595                 600                 605
   Ser Tyr Glu Asn Asn Asn Arg Tyr Gly Arg Tyr Leu Gln Tyr Gly Gln
       610                 615                 620
   Leu Glu Ile Ile Pro Ala Asp Leu Thr Gln Ser Gly Phe Ser His Ala
   625                 630                 635                 640
   Gly Trp Asp Trp Asn Arg Tyr Pro Gly Thr Thr Thr Ile His Leu Pro
                   645                 650                 655
   Tyr Asn Glu Leu Glu Ala Lys Leu Asn Gln Leu Pro Ala Ala Gly Ile
               660                 665                 670
   Glu Glu Met Leu Leu Ser Thr Glu Ser Tyr Ser Gly Ala Asn Thr Leu
           675                 680                 685
   Asn Asn Asn Ser Met Phe Ala Met Lys Leu His Gly His Ser Lys Tyr
       690                 695                 700
   Gln Gln Gln Ser Leu Arg Ala Asn Lys Ser Tyr Phe Leu Phe Asp Asn
   705 710 715 720
   Arg Val Ile Ala Leu Gly Ser Gly Ile Glu Asn Asp Asp Lys Gln His
   725 730 735
   Thr Thr Glu Thr Thr Leu Phe Gln Phe Ala Val Pro Lys Leu Gln Ser
   740 745 750
   Val Ile Ile Asn Gly Lys Lys Val Asn Gln Leu Asp Thr Gln Leu Thr
   755 760 765
   Leu Asn Asn Ala Asp Thr Leu Ile Asp Pro Ala Gly Asn Leu Tyr Lys
   770 775 780
   Leu Thr Lys Gly Gln Thr Val Lys Phe Ser Tyr Gln Lys Gln His Ser
   785 790 795 800
   Leu Asp Asp Arg Asn Ser Lys Pro Thr Glu Gln Leu Phe Ala Thr Ala
   805 810 815
   Val Ile Ser His Gly Lys Ala Pro Ser Asn Glu Asn Tyr Glu Tyr Ala
   820 825 830
   Ile Ala Ile Glu Ala Gln Asn Asn Lys Ala Pro Lys Tyr Thr Val Leu
   835 840 845
   Gln His Asn Asp Gln Leu His Ala Val Lys Asp Lys Ile Thr Gln Glu
   850 855 860
   Glu Gly Tyr Gly Phe Phe Glu Ala Thr Lys Leu Lys Ser Ala Asp Ala
   865 870 875 880
   Thr Leu Leu Ser Ser Asp Ala Pro Val Met Val Met Ala Lys Ile Gln
   885 890 895
   Asn Gln Gln Leu Thr Leu Ser Ile Val Asn Pro Asp Leu Asn Leu Tyr
   900 905 910
   Gln Gly Arg Glu Lys Asp Gln Phe Asp Asp Lys Gly Asn Gln Ile Glu
   915 920 925
   Val Ser Val Tyr Ser Arg His Trp Leu Thr Ala Glu Ser Gln Ser Thr
   930 935 940
   Asn Ser Thr Ile Thr Val Lys Gly Ile Trp Lys Leu Thr Thr Pro Gln
   945 950 955 960
   Pro Gly Val Ile Ile Lys His His Asn Asn Asn Thr Leu Ile Thr Thr
   965 970 975
   Thr Thr Ile Gln Ala Thr Pro Thr Val Ile Asn Leu Val Lys
   980 985 990

## Description

### BACKGROUND OF THE INVENTION

### Field of the Invention

The present invention generally relates to methods for promoting neurological functional recovery after central nervous system ("CNS") injury or disease. In particular, the present invention is directed to a method of utilizing chondroitinase to promote autonomic neurological functional recovery following injury in or to the spinal cord. Compositions useful in this method include acceptable formulations of chondroitinase, more particularly sustained release formulations of chondroitinase. The present invention is also directed to a method of promoting neurological functional recovery after a contusion injury to the spinal cord.

### Description of Related Art

The spinal cord is the largest nerve in the body and is made up of nerve fibers. These nerve fibers are responsible for the body's communication systems, which include sensory, motor, and autonomic functions. Sensory functions include the ability to feel sensations, like pain. Motor function include the ability to voluntarily move your body. Autonomic functions include involuntary body functions, for example the ability to sweat and breathe.

The central nervous system includes the brain and spinal cord. The spinal cord connects the peripheral nervous system ("PNS') to the brain. Sensory nerves, which enter the dorsal root of the spinal cord; transmit sensory information from the sensory receptors of the body to the brain. Different types of sensation are sent in different sensory pathways. For example, the spinothalamic tracts carry sensations of pain and temperature and dorsal column tracts carry sensations of position and touch. Motor nerves, which exit the ventral root nerves of the spinal cord, transmit voluntary motor information from the brain to the body.

The autonomic nervous system (ANS) influences the activities of involuntary muscles, include the heart muscle, and glands that release hormones. In particular, the ANS controls the cardiovascular, digestive and respiratory systems to maintain a stable environment within the body. The ANS includes sympathetic nerves, that cause constriction of the blood vessels and increased heart rate, and parasympathetic nerves, which act in an opposite manner to the sympathetic nerves by dilating blood vessels and decreasing heart rate.

Damage to the central nervous system, including the spinal cord, results in a loss of function. Depending upon the type of injury to the central nervous system, the loss of function may manifest itself in loss of sensory, motor or autonomic function or a combination thereof.

The most common types of spinal cord injuries (SCI) include contusions (bruising of the spinal cord) and compression injuries (caused by pressure on the spinal cord). In contusion injuries, the most common type of injury, a cavity or hole often forms in the center of the spinal cord. Unlike nerve cells, or neurons of the PNS, neurons of the CNS do not regenerate after injury. The inability of axons to regenerate may lead to loss of sensation, motor function and autonomic function, as well as permanent paralysis. One reason that neurons fail to regenerate may be their inability to traverse the glial scar that develops following a spinal cord injury. The injury- caused lesion will develop glial scarring, which contains extracellular matrix molecules including chondroitin sulfate proteoglycans (CSPGs). CSPGs inhibit nerve tissue growth *in vitro* and nerve tissue regeneration at CSPGs rich regions *in vivo.*

A number of molecules, and specified regions thereof, have been implicated in the ability to support the sprouting of neurites from a neuronal cell, a process also referred to as neurite outgrowth. The term neurite refers to both axon and dendrite structures. This process of sprouting neurites is essential in neural development and regeneration, especially after physical injury or disease has damaged neuronal cells. Neurites elongate profusely during development both in the central and peripheral nervous systems of all animal species. This phenomenon pertains to both axons and dendrites.

Various polypeptides, especially cell adhesion molecules (CAMs), have been known to promote neural cell growth. While early efforts in this area of research concentrated on the adhesion-promoting extracellular matrix protein fibronectin (FN), other polypeptides have also been found to promote neural growth. For example, U.S. Patent No. 5,792,743 discloses novel polypeptides and methods for promoting neural growth in the central nervous system of a mammal by administering a soluble neural CAM, a fragment thereof, or a Fc-fusion product thereof. U.S. Patent No. 6,313,265 discloses synthetic polypeptides containing the pharmacologically active regions of CAMs that can be used in promoting nerve regeneration and repair in both peripheral nerve injuries as well as lesions in the central nervous system. While helpful, the use of regenerative proteins alone may not be sufficient to effect repair of a damaged nervous system. WO 03/04080 describes use of glycosaminoglycan degrading enzymes for treating injured cortecospinal tract, but does not reference contusion injuries.

During approximately the past two decades, the base knowledge of cell adhesion and migration in extracellular matrices (ECMs) at the molecular level has expanded rapidly. The action of enzymes and other polypeptides which degrade components of the extracellular matrix and basement membranes may facilitate the events of neural repair by a variety of mechanisms including the release of bound cytokines and by increasing the permeability of the matrix, thereby enhancing the mobility of mediator molecules, growth factors and chemotactic agents, as well as the cells involved in the healing process. For example, U.S. Patent No. 5,997,863 discloses the use of glycosaminoglycans to manipulate cell proliferation and promote wound healing.

ECM molecules include the inhibitory CSPGs. Components of the CSPGs have been identified as the glycosaminoglycans, chondroitin sulfate (CS) and dermatan sulfate (DS). Removal of these inhibitory molecules would allow neurites to regenerate and reinnervate an area after physical injury or disease, as well as allow for recovery of sensory, motor and autonomic functions.

Previous studies have found that chondroitinases can lyse and degrade CSPGs including, CS and DS. One study found that chondroitinase ABC removed glycosaminoglycan (GAG) chains in and around lesioned areas of rat CNS *in vivo.* The degradation of GAGs promoted expression of a growth-associated protein, GAP-43, indicating increased regenerative propensity in treated cells. However, this growth-associated protein is associated with regeneration in peripheral, but not central, nerve injuries. Another study found that *in vitro* chondroitinase ABC treatment of rat spinal cord regenerated neurons on a tissue section substrata. This study observed that degradation of CSPGs may promote the neuro-stimulatory effects of laminin. (Zuo et al. Degradation of chondroitin sulfate proteoglycan enhances the neurite- promoting potential of spinal cord tissue, Exp. Neurol. 154(2): 654-62 (1998)). In a later study by the same primary researcher, it was reported that injection of chondroitinase ABC at the site of nerve damage degraded CSPGs and increased the ingress of axonal sprouts into the basal laminae of the distal nerve segment, which may be enabling more latitude in growth at the interface of coapted nerve. (Zuo et al. Regeneration of axons after nerve transaction repair is enhanced by degradation of chondroitin sulfate proteoglycan. Exp. Neurol. 176(1): 221-8 (2002)). The same group of researchers also found chondroitinase ABC treatments regenerated axons into acellular grafts at a much higher rate than the control grafts. (Krekoski et al., Axonal regeneration into acellular nerve grafts is enhanced by degradation of chondroitin sulfate proteoglycan. J. Neurosci. 15:21(16): 6206-13 (2001)). Applications of chondroitinase ABC_{TypeI} to an injured corticospinal tract, in particular, an injury to the dorsal column, prevented axon retraction from the affected area and promoted more axon fiber growth than the control, with some axons arborizing into gray matter. Regenerated CST axons established functional connections. (Bradbury et al., Chondroitinase ABC promotes functional recovery after spinal cord injury, Nature 416: 636- 640 (2002)).

However, chondroitinase-induced neurological functional recovery in a dorsal column transection lesion animal model has limited applicability or predictive power relative to recovery of autonomic function, and in particular, as a result of a contusion injury in the spinal cord. In the dorsal column transaction lesion described by Bradbury et al 2002, dorsal column fibers are lesioned by cutting the nerve tracts with a knife. This method produces a localized or "clean" lesion that severs the nerve fibers with minimal collateral damage to the remaining parenchyma of the spinal cord. Gray matter tissue and other white matter tracts sustain minimal damage, and thus.this model is useful for studying the regenerative capacity of the dorsal column neurons, which carry sensory neurons.

### SUMMARY OF THE INVENTION

The present invention is generally directed to for use in improving functional recovery following contusion injury of the CNS, because these chondroitinases degrade components of the CNS extracellular matrix that are inhibitory to regeneration.

The chondroitinases can be administered to a mammal afflicted with a CNS injury, whether the injury is immediate or long-standing. The chondroitinase is administered in an amount effective to degrade CSPGs and thereby promote the recovery of autonomic neurological function.

The chondroitinases optimally may be administered with a suitable pharmaceutical carrier. The administration can be local or systemic, including oral, parenteral, intraperitoneal, intrathecal or topical application. The release profiles of such formulations may be rapid release, immediate release, controlled release or sustained release. For example, the formulation may comprise a sustained release matrix and a therapeutically effective amount of a glycosamino-glycan degrading enzyme. Alternatively, chondroitinases can be secreted by genetically modified cells that are implanted, either free or in a capsule, at or near the site of CNS injury.

The administration of chondroitinases and the resulting promotion of neurological functional recovery in accordance with this disclosure restores motor, sensory and autonomic functions, to varying degrees, depending on the responsiveness of each individual following contusive or non-contusive injury to the central nervous system.

### BRIEF DESCRIPTION OF THE DRAWINGS

For a fuller understanding of the nature and advantages of the present invention, reference should be had to the following detailed description taken in connection with the accompanying drawings, in which:

Figure 1 is a graphical illustration of residual urine volumes following contusive spinal cord injury.

Figure 2 is a graph of the release rate of chondroitinase from the sustained release matrices.

Figure 3 is a graph of the BBB scores of rats following treatment with chondroitinase ABC_{Type 1}, penicillinase or control following contusive spinal cord injury.

Figure 4 is a graph of the BBB scores of rats following treatment with chondroitinase ABC_{Type 1} or penicillinase following contusive spinal cord injury.

Figure 5 is a graph of the mean changes in body weight following administration of varying doses of chondroitinase.

Figure 6 is a graphical illustration of the mean weight change by dose in varying doses of Chondroitinase ABC _{Type 1}.

Figure 7 is a graphical illustration of the mean temperature change by in varying doses of Chondroitinase ABC _{Type 1.}

Figure 8 is a graphical illustration of the weight change in repeat and escalating doses of Chondroitinase ABC _{Type 1}.

Figure 9 is a graphical illustration of the temperature change in repeat and escalating doses of Chondroitinase ABC _{Type 1}.

### DETAILED DESCRIPTION

Before the present compositions and methods are described, it is to be understood that this invention is not limited to the particular processes, compositions, or methodologies described, as these may vary. It is also to be understood that the terminology used in the description is for the purpose of describing the particular versions or embodiments only, and is not intended to limit the scope of the present invention which will be limited only by the appended claims.

It must also be noted that as used herein and in the appended claims, the singular forms "a", "an", and "the" include plural reference unless the context clearly dictates otherwise. Thus, for example, reference to an "enzyme" is a reference to one or more enzymes and equivalents thereof known to those skilled in the art, and so forth. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described. Nothing herein is to be construed as an admission that the invention is not entitled to antedate such disclosure by virtue of prior invention.

The present disclosure is directed to a chondroitinase ABC enzyme for use in improving functional recovery following a contusion injury of the CNS, typically caused by trauma or disease. In particular, chondroitinase ABC_{Type I} or, chondroitinase ABC_{Type II} individually and in combination, provide a therapeutic treatment for spinal cord injuries. The phrase "contusion injury" as used herein includes disease and traumatic injuries that may result in tearing, bruising or crushing of neurons brought about by a traumatic injury, such as an auto accident, fall, or bullet wound, as well as other injuries, or a non-traumatic injury, or compression that may be caused from internal or external sources. Practice of the present methods will confer clinical benefits to the treated mammal, providing clinically relevant improvements in at least one of the subject's motor coordination functions, sensory perception, or autonomic functions. Clinically relevant improvements can range from a detectable improvement to a complete restoration of an impaired or lost function of the CNS.

In contrast to the "clean" lesions described by Bradbury, the contusion model of spinal cord injury produces an indiscriminate destruction of the tissue through a cascade of response mechanisms. A contusion injury is produced experimentally by applying a blunt force to the exposed spinal cord and more accurately mimics the typical human injury, providing a more suitable condition for the study of both primary and secondary pathophysiologic processes (Blight AR. 2000 Animal models of spinal cord injury. Top Spinal Cord Inj Rehabil 6(2):1-13; Kwon BK. Oxland TR, Tetzlaff W. (2002) Animal models used in spinal cord regeneration research. Spine 27(14):1504-10)). Following the immediate mechanical damage to the tissue, which can stretch and tear at axons in the white matter, a more profound and general destruction of the tissue ensues. The blood-brain barrier is compromised and the spinal cord undergoes a central gray matter hemorrhagic necrosis triggering inflammatory and biochemical cascades that result in extensive secondary cell and tissue damage. In a period of days to weeks a cavitated lesion forms at the injury site that may be filled with reactive glial cells, residual axons, neovasculature and a deposition of extracellular matrix molecules. Sensory, motor and autonomic function may be compromised. Compared to the relatively "clean" nature of a dorsal column transection lesion, a contusive injury produces a much more complicated injury that involves the repertoire of response and repair mechanisms of the spinal cord tissue and is appropriate for evaluating potential therapeutics that target both the acute and chronic phase of injury. Additionally, contusion injuries result in secondary effects resulting in tissue loss, scarring, cavity formation and the like. Contusion injury triggers a more robust immune response compared to transection injury, which may be of significant consequence for injury and repair (Hirschberg DL, Yoles E, Belkin M, Schwartz M. 1994).

The experimental paradigm in which to evaluate a potential therapeutic for SCI must be placed into context when transposing results onto other models of spinal cord injury. The contrast between the anatomy and pathophysiology of the dorsal column transection injury and the contusion model are extensive. Dorsal column transection injuries cause damage to neurons that are more prone to regenerate and have specific functions within the sensory tracts. Furthermore, transection injury leads to minimal secondary tissue damage. In contrast, contusion injury damages both sensory and motor nerve tracts, affective sensory, motor and autonomic function. The corticospinal tracts are far less regenerative than the neurons found in the dorsal columns. Contusion injury also leads to extensive secondary tissue damage that creates a lesion substantially larger than a dorsal column lesion.

After a spinal cord injury in the adult mammalian CNS, the inability of axons to regenerate may lead to permanent paralysis. The site of the CNS injury develops a lesion or glial scar by an increase in the deposition of extracellular matrix molecules by astrocytes and oligodendrocytes at the site of injury. These extracellular matrix molecules include CSPGs, which are highly expressed in scarring areas. CSPGs inhibit nerve tissue growth *in vitro,* and nerve tissue regeneration at CSPGs rich regions *in vivo.* Chondroitin sultates A, B and C are the predominant forms found in mammals. These chondroitins may be involved in modulation of various biological activities including cell differentiation, adhesion, enzymatic pathways, and hormone interactions. The presence of chondroitin sulfate proteoglycans is elevated in the later stages of cell growth in response to tissue and vessel damage.

The glycosaminoglycans (GAGs), chondroitin sulfate (CS) and dermatan sulfate (DS), are important components of CSPG. They are inhibitory molecules that contribute to the lack of regeneration of the CNS in adult mammals, by hindering axonal and neuritic growth. However, CSPGs are important in neuronal guidance and patterning during development, rather than inhibition.

Glycosaminoglycans are unbranched polysaccharides consisting of alternating hexosamine and hexuronic residues which carry sulfate groups in different positions. The GAGs are typically divided into three families according to the composition of the disaccharide backbone. These are: heparin/heparan sulfate; chondroitin sulfate; and keratan sulfate. The chondroitin sulfate family includes seven sub-types designated unsulfated chondroitin sulfate, oversulfated chondroitin sulfate, and chondroitin sulfates A-E, which vary in the number and position of their sulfate functional groups. Chondroitin sulfate B is also referred to as dermatan sulfate, and it differs in that iduronic acid is the predominant residue in the alternative hexuronic acid position.

It has now been found that the CSPG-degrading enzymes such as chondroitinase ABC_{TypeI} or chondroitinase ABC_{TypeII}
are useful in controlling and/or inhibiting the effects of chondroitin sulfates and in developing therapeutics for the treatment of disease states. The findings described herein are the first description of chondroitinase treatment of contusion injury causing an improvement in the recovery of neurological function, in particular autonomic function, following contusion injury of the spinal cord.

Chondroitinase AC and chondroitinase B are chondroitin lyase enzymes, which may be derived from various sources. Any chondroitinase AC or B may be used in the disclosure, including, but not limited to chondroitinase AC (derived from Flavobacterium heparinum; T. Yamagata, H. Saito, O. Habuchi, S. Suzuki, J. Biol. Chem., 243, 1523 (1968)); chondroitinase AC II (derived for Arthobacter aurescens; K. Hiyama, S. Okada, J. Biol. Chem., 250, 1824 (1975), K. Hiyama, S. Okada, J. Biochem. (Tokyo), 80, 1201 (1976)); chondroitinase AC III (derived from Flavobacterium sp. Hp102; H. Miyazono, H. Kikuchi, K. Yoshida, K. Morikawa, K. Tokuyasu, Seikagaku, 61, 1023 (1989)); chondroitinase B (derived from Flavobacterium heparinum; Y. M. Michelaaci, C.P. Dietrich, Biochem. Biophys. Res. Commun., 56, 973 (1974), V. M. Michelaaci, C.P. Dietrich, Biochem. J., 151, 121 (1975), K. Maeyama, A. Tawada, A. Ueno, K. Yoshida, Seikagaku, 57, 1189 (1985)); and chondroitinase B (derived from Flavobacterium sp. Hp102; H. Miyazono, H. Kikuchi, K. Yoshida, K. Morikawa, K. Tokuyasu, Seikagaku, 61, 1023 (1989)). Chondroitinase AC and chondroitinase B are commercially available from Seikagaku America, Falmouth, Massachusetts, USA. Additionally, the enzymes may be produced by the methods disclosed in U.S. Patent No. 6,093,563 by Bennett et al. Chondroitinase ABC_{TypeI} and chondroitinase ABC_{TypeII} are exo- and endo-lyases respectively which cleave both chondroitin and dermatan sulfates (Hamei et al 1997). Mammalian enzymes with chondroitinase-like activity have been identified.

Chondroitinase enzyme activity can be stabilized by the addition of excipients or by lyophilization. Stabilizers include carbohydrates, amino acids, fatty acids, and surfactants and are known to those skilled in the art. Examples include carbohydrate such as sucrose, lactose, mannitol, and dextran, proteins such as albumin and protamine, amino acids such as arginine, glycine, and threonine, surfactants such as TWEEN® and PLURONIC®, salts such as calcium chloride and sodium phosphate, and lipids such as fatty acids, phospholipids, and bile salts. The stabilizers are generally added to the protein in a ratio of 1:10 to 4:1, carbohydrate to protein, amino acids to protein, protein stabilizer to protein, and salts to protein; 1:1000 to 1:20, surfactant to protein; and 1:20 to 4:1, lipids to protein. Other stabilizers include high concentrations of ammonium sulfate, sodium acetate or sodium sulfate, based on comparative studies with heparinase activity. The stabilizing agents, preferably the ammonium sulfate or other similar salt, are added to the enzyme in a ratio of 0.1 to 4.0 mg ammonium sulfate/IU enzyme.

Chondroitinase may be administered locally or systemically. Such administration includes oral, parenteral, enteral, intraperitoneal, intrathecal, inhalation, or topical administration. The preferred forms of administration include intravenous, subcutaneous, intrathecally, intradermal, intramuscular, internodal, intracutaneous, or percutaneous. Topical or local administration may preferable for greater control of application.

The chondroitinases, singularly or in combination, can be mixed with an appropriate pharmaceutical carrier prior to administration. Examples of generally used pharmaceutical carriers and additives are conventional diluents, binders, lubricants, coloring agents, disintegrating agents, buffer agents, isotonizing fatty acids, isotonizing agents, preservants, anesthetics, surfactants and the like, and are known to those skilled in the art. Specifically pharmaceutical carriers that may be used are dextran, sucrose, lactose, maltose, xylose, trehalose, mannitol, xylitol, sorbitol, inositol, serum albumin, gelatin, creatinine, polyethlene glycol, non-ionic surfactants (e.g. polyoxyethylene sorbitan fatty acid esters, polyoxyethylene hardened castor oil, sucrose fatty acid esters, polyoxyethylene polyoxypropylene glycol) and similar compounds. Pharmaceutical carriers may also be used in combination, such as polyethylene glycol and/or sucrose, or polyoxyethylene sorbitan fatty acid esters, polyoxyethylene sorbitan monooleate (20 E. 0.) is particularly preferred.

The release profiles of such formulations may be rapid release, immediate release, controlled release or sustained release. In particular, sustained release formulations of or chondroitinase ABC_{TypeI} or chondroitinase ABC_{TypeII} may be used to improve or recover neurological function, including autonomic function. Such a formulation results in a controlled, sustained release of the enzyme into the system such that CSPGs are degraded. Degradation of CSPGs may occur at the site of injury, cavity or lesion or may occur at sites within the CNS upstream or downstream of the injury.

The treatment regimen according to the invention may be carried out by a means of administering chondroitinase ABC_{TypeI} or chondroitinase ABC_{TypeII} preferably to the CNS, and more preferably to the lesions of the injured area of the CNS. The mode of administration, the timing of administration and the dosage are carried out such that the functional recovery from impairment of the CNS is enhanced by the promotion of neurite outgrowth. The treatments of the present disclosure deliver an effective amount of chondroitinase ABC_{TypeI} or chondroitinase ABC_{TypeII} The term "effective amount" means an amount sufficient to degrade the CSPGs of the lesioned area of the spinal cord or within the CNS or an amount sufficient to restore, in whole or in part, motor, sensory or autonomic function of the mammal. For example, an effective amount of enzyme may be about .0001 mg/kg to about 100 mg/kg body weight of the patient. The effective amount of chondroitinase can be administered in a single dosage, two dosages or a plurality of dosages. Although it is to be understood that the dosage may be administered at any time, in one embodiment, the dosage is administered within 12 hours after injury, or as soon as is feasible. In another embodiment, the dosage is administered to an injured mammal in one, two or a plurality of dosages; such dosages would be dependant on the severity of the injury and the amount of CSPGs present in the glial scarring. Where a plurality of dosages is administered, they may be delivered on a daily, weekly, or biweekly basis. The delivery of the dosages may be by means of catheter or syringe. Alternatively, the treatment can be administered during surgery to allow direct application to the glial scar.

Once the chondroitinases are administered, the degradation of CSPGs removes the inhibitory molecules that block neurite outgrowth, and allow the regeneration of neurites into the affected area. Administration of chondroitinases may also degrade CSPGs in the CNS distant to the affected area, including upstream or downstream of the injury. Chondroitinase ABC_{TypeI}, chondroitinase ABC_{TypeII}, are exo- and endo-lyases that cleave both CS and DS. The removal of CS and DS from the glial scar permits the regeneration of neurite outgrowths into the injured area.

The regeneration of the nerve cells in to the affected CNS area allows the return of motor, sensory and autonomic function. Clinically relevant improvement will range from a detectable improvement to a complete restoration of an impaired or lost nervous function, varying with the individual patients and injuries.

Practice of the invention, including additional preferred aspects and embodiments thereof, will be more fully understood from the following examples, which are presented for illustration only and should not be construed as limiting the invention in any way.

### Example 1:

### Chondroitinase improves autonomic functions following injury of the spinal cord in rodents

A study of autonomic function following contusion injury of the spinal cord in rats with the use of Chondroitinase was completed in the Acorda Animal Modeling Facility. Animals (n=38) were subjected to an established model of SCI (Gruner et al. 1996). Beginning immediately following the SCI, 19 animals were treated with Chondroitinase ABC 1 (Seikagaku; Cat number 100332, lot number E02201) intrathecally (i.t.) at 0.06 Units per rat per dose in artificial cerebrospinal fluid, every other day for two weeks. The other 19 animals were treated with enzymatic protein (Penicillinase - Sigma; Cat number P4524) in vehicle.

The animals were induced and maintained in a state of surgical anesthesia with 1.5% isoflurane carried in medical grade air (95% oxygen, 5% CO₂) mixture. An initial dose of Cefazolin (50 mg/kg, s.c.) was given preoperative. During surgery, the animal were placed on a heating pad to help maintain body temperature, and the pulse rate, SpO2 and temperature of the animal were monitored. A laminectomy of the T9 and T10 spinal vertebrae was performed. A partial laminectomy at the T13 / L1 junction was made for the placement of an intrathecal catheter. An incision in the dura was made with a hypodermic needle. A 32 gauge catheter (ReCathCo.. LLC, CS132G, lot number 20422) was inserted through the dural incision and fed rostally to lie immediately caudal to the T9 / T10 laminectomy. The catheter was secured to bone and muscle with cyanoacrylate glue and sutures. The blades of modified coverslip forceps (4 mm wide x 0.5 mm thick) were inserted into the spinal canal between the lateral aspects of the spinal cord and vertebrae at the rostral laminectomy. A SCI was induced by compressing the spinal cord between the blades of the forceps for a period of 15 seconds. The level of injury severity was induced by using forceps that compress the spinal cord to a distance of 0.3 mm (moderate injury). The dura over the injury remains intact. The forceps were removed, and the injury site flushed with saline. Overlaying muscle layers were sutured together and the skin wound was stapled closed. Post operatively, the animals were given an immediate 5 ml bolus of lactated Ringer's saline solution followed by a second administration of 5 mls lactated Ringer's saline solution after a few hours.

Immediately after surgery and then every other day for 2 weeks, animals were anesthetized with isoflurane and the experimental (Chondoitinase ABC I) or control agent (Penicillinase) as described below were injected into the intrathecal catheter. The volume injected was 3 microliters, followed by a 4 microliter wash of artificial cerebrospinal fluid.

### Treatment Regimens:

1. Chase ABC 1 - Chondroitinase ABC 1 (Seikagaku), 0.06 U / dose, i.t. in 3 microliters aCSF.
2. Penicillinase - Penicillinase (Sigma), 3 microliters, i.t. at 228 micrograms per milliliter.

### Analysis and Results:

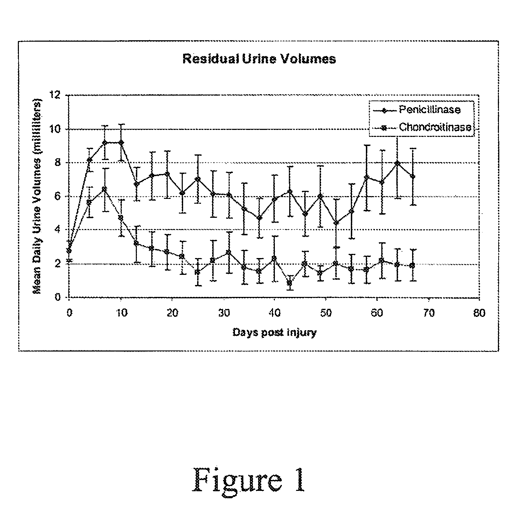

Urinary bladders were manually expressed at least twice daily and urine volumes recorded. Urine volumes in all groups were about 2.7 milliliters for the first 4 days following injury. Urine volumes in the penicillinase treated group reached peak volumes of 11 milliliters, returning to between 6 and 8 milliliters per day by about 3 weeks post injury. Urine volumes in the chondroitinase treated group reached maximal daily volumes of 6 milliliters, returning to about 2 milliliters by 3 weeks. See Figure 1 for a plot of mean residual urine volumes from the moderate injury, group from rats treated with chondroitinase and penicillinase. This system is well accepted in the field for the assessment of recovery of autonomic function following spinal cord injury in the rat.

These results demonstrate the potential use of a chondroitinase enzyme for the recovery of autonomic function following spinal cord injury. The spinal cord injury model and behavioral analysis system are well accepted in the field are believed to be the most relevant to most human spinal cord injuries.

### Example 2

### Sustained release formulations of Chondroitinase

The development of a sustained release chondroitinase enzyme delivery technology allows chondroitinase to be administered at any point following SCI, for a given duration. An ideal sustained release system for chondroitinase is one that not only affords prolonged release of the active agent, but one that is practical to use in the context of SCI. At a minimum, the design criteria includes biocompatibility of the device in the CNS, retention of chondroitinase catalytic activity and appropriate chondroitinase release kinetics. Preferably, the system is in the form of a thin film that is applied to the site of SCI or a polymerizing system that is applied to the site, polymerize on contact with the spinal cord and then stay in place throughout the course of the treatment period. The system is pliant so that introduction to the SCI does not lead to additional trauma in the form of compression.

Over the last few years advancements in biodegradable drug delivery systems have resulted in a variety of viable matrix candidates. The selection of an ideal system depends on an evaluation of the biochemical and practical attributed. In general, these systems capture the active agent in a matrix that is held together in a uniform phase through formation of covalent cross-linking, a crystalline matrix, an emulsion or a phase transition. The matrix material are either a biological system or a synthetic polymer. Examples of the candidate systems include biological matrices like fibrin glue, collagen and alginate and examples of synthetic matrices include polylactic/polyglycolic acid, pluronic and ethylene vinyl acetate. It is to be understood that sustained release formulations herein are not only suitable for use in the treatment of contusion injuries to the CNS, but also are suitable for use in the treatment of other CNS injuries and disorders.

Recombinant Chase enzymes developed may be loaded into a variety of SR matrices, including, but not limited to fibrin glue, collagen, alginate, polyactic/polyglycolic acid, pluronic and ethylene vinyl acetate. Both natural and synthetic polymers may be used. Synthetic polymers have the advantage of precise formulation and predictable release properties, while natural polymer preparations may have increased biocompatibility. Each type of polymer system has unique properties and the methods to generate Chase impregnated films or gels for each type is described below:

Collagen Gels: Collagen has been used as a biomaterial in medical devices for decades because of its ease of preparation and biocompatibility. Collagen is found in approved medical devices such as hemostats, tissue augmentation gels, bone graft substitutes, sealants and a variety of wound healing products. The collagen used for biomedical purposes is typically fibrilar type I collagen isolated from bovine tendons. It can be fashioned into a variety of forms including gels, films and fibers. Collagen gels may be formed from rodent type I collagen. The formation methods for collagen gels have been used routinely for many years. Collagen in dilute acid is combined with a solution of Chase in a buffer at physiological pH. When neutralized and warmed the type I collagen fibrils coalesce into disordered arrays to create a gel. The crosslinking density of the resulting gels may be controlled by varying the concentration of collagen.

Fibrin-Glue: Fibrin glue is a blood-derived product that has been used clinically in Europe for many years as a hemostat or sealant. Fibrin glue consists of two components that are combined to form a gel. The first constituent is fibrinogen which is the major protein involved in clotting. Fibrinogen is combined with thrombin to recapitulate the final step in the clotting cascade. Thrombin is a serine protease that acts on fibrinogen to expose reactive termini that drive polymerization of fibrinogen into a fibrous network called fibrin. Chase may be combined with the fibrinogen solution and then polymerized by the addition of thrombin.

Alginate: Alginate is a large molecular weight carbohydrate isolated from marine kelp. It is most often used as an additive in foods and cosmetics. Its safety and biocompatibility have resulted in its use in wound healing products. It is also used in experimental preparations for the microencapsulation of cells or pancreatic islets. Alginate is particularly versatile because it can be formed into a variety of shapes and forms via ionic crosslinking with divalent cations such as calcium. Crosslinking density can be controlled by the concentration of the calcium and alginate and the resulting gels are stable for long periods of time *in vivo.* A solution of Chase and alginate may be used to form films upon the addition of calcium.

PLA/PGA: Polylactic acid and Polyglycolic polymers or copolymers are hydrolytically unstable polyesters used in sutures and other biodegradable implantable medical devices. PLA/PGA have also been used to create microcapsules for drug delivery. There are several methods available to create sustained release systems with these polymers. For example, a solvent exchange system may be used wherein PLA/PGA polymers are dissolved in n-methylpyrrolidone.

Pluronic: Pluronics are hydrophilic polymers that undergo a reverse phase transition. Pluronic solutions are viscous liquids at low temperatures and then undergo a liquid to solid phase transition when shifted to warmer temperatures. Pluronics have been used in a variety of biomedical applications where it is desirable to inject or spray a liquid on a tissue and then have the liquid solidify into a film. Pluronics may be dissolved in the aqueous buffers containing Chase. Films may be created by casting gels in tissue culture plates that are warmed in an incubator to promote the reverse phase transition and film formation.

Ethylene vinyl acetate: Ethylene vinyl acetate (EVA) is representative of polymers used to make nondegradable, biocompatible implants. EVA is soluble in certain organic solvents such as methylene chloride. Chase in an aqueous buffer may be added to the EVA solution under agitation to create an emulsion. The organic solvent may then be evaporated from the solution which drives the formation of a semi-crystalline polymer matrix impregnated with Chase. This method may be used to make a range of crosslinking densities.

### Example 3:

### Sustained release formulations of Chondroitinase ABC_{Type I}

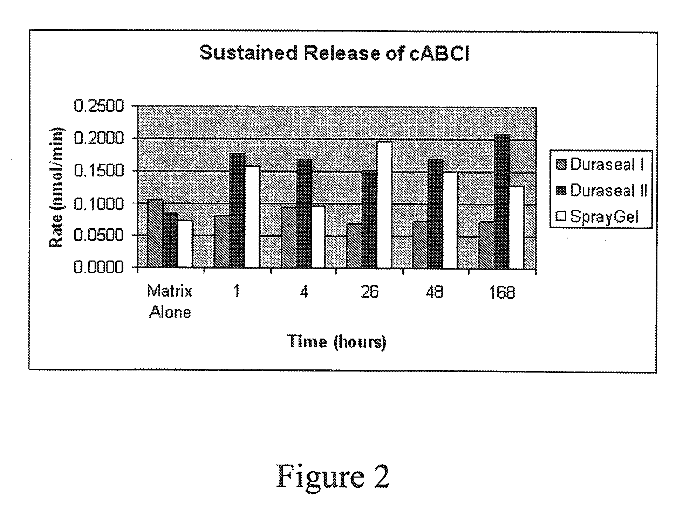

In one study, Chondroitinase ABC_{Type I} was formulated into three sustained release matrices: Duraseal™ I (available from Confluent Surgical), Duraseal™ II (available from Confluent Surgical) and Spray Gel. Duraseal™ is an augmented hydrogel. The Spray Gel is a collagen based gel foam. Release was monitored by measuring chondroitinase activity released from the matrices over time. Results are illustrated in Figure 2. The results demonstrate that chondroitinase is released from a sustained release matrix over time and may be formulated in a sustained release formulation.

### Example 4:

### Chondroitinase ABC_{TypeI} improves neurological function following contusion injury of the spinal cord in rodents

A study of chondroitinase ABC_{TypeI} treatment of a contusion injury in the rat was completed in the Acorda Animal Modeling Facility. Animals (n=30) were subjected to an established model of SCI (Gruner et al. 1996). Beginning immediately following the SCI, ten animals were treated with chondroitinase ABC I (Seikagaku; Cat number 100332, lot number E02201) intrathecally (i.t.) at 0.06 Units per rat per dose in artifical cerebrospinal fluid, every day for one week and then on alternating days for one week. Another ten animals received enzymatic protein (Penicillinase - Sigma; Cat number P4524) and another ten animals received vehicle control (artifical cerebrospinal fluid - Harvard Apparatus; Cat number 59-7316). Animals were evaluated by open-field behavioral testing for a period of 12 to 16 weeks.

The animals were induced and maintained in a state of surgical anesthesia with 1.5% isoflorane carried in medical grade air (95% oxygen, 5% CO₂) mixture. An initial dose of Baytril (25 mg/kg) was given preoperative. During surgery, the animal were placed on a heating pad to help maintain body temperature, and the pulse rate, SpO2 and temperature of the animal. A laminectomy of the T9 and T10 spinal vertebrae was performed. A partial laminectomy at the T13 / L1 junction was made for the placement of an intrathecal catheter. An incision in the dura was made with a hypodermic needle. A catheter (Harvard Apparatus) was inserted through the dural incision and fed rostally to lie immediately caudal to the T9 / T10 laminectomy. The catheter was secured to bone and muscle with cyanoacrylate glue and sutures. The blades of modified coverslip forceps (4 mm wide x 0.5 mm thick) were inserted into the spinal canal between the lateral aspects of the spinal cord and vertebrae at the rostral laminectomy. A SCI was induced by compressing the spinal cord between the blades of the forceps for a period of 15 seconds. The forceps are designed to compress the spinal cord to a distance of 0.9 mm. The forceps were removed, and the injury site flushed with saline. Overlaying muscle layers were sutured together and the skin wound was stapled closed. Post operatively, the animals were given an immediate 5 ml bolus of lactated Ringer's saline solution followed by a second administration of 5 mls lactated Ringer's saline solution after a few hours.

Immediately after surgery and then every day for 1 week and then on alternating days for 1 week, animals were anesthetized with isoflurane and the experimental or control agents (chondroitinase ABC 1, penicillinase or aCSF) as described below was injected into the intrathecal catheter. The volume injected was 6 microliters, followed by a 6 microliter wash of artifical cerebrospinal fluid.
Treatment Regimens:
1. Chase ABC 1 - Chondroitinase ABC 1 (Seikagaku), 0.06 U / dose, i.t. in 6 microliters aCSF.
2. Penicillinase - Penicillinase (Sigma), 6 microliters, i.t. at 124 micrograms per milliliter.
3. aCSF - artifical cerebrospinal fluid (Harvard Apparatus), 6 microliters, i.t. Behavioral Analysis:

At 48 hours and then weekly after injury, open field locomotor activity was observed and scored according to the Basso, Beattie and Bresnahan (BBB) scoring system (Basso et al., 1995). This system is well accepted in the field for the assessment of recovery of locomtor function following spinal cord injury in the rat.

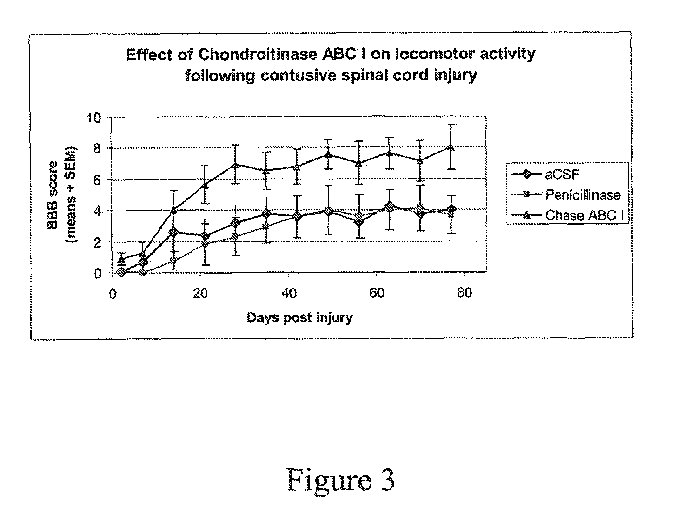

Results: There was a mortality rate of 40% evenly distributed across all of the treatment groups that was due to the catheterization and adverse events associated with the severity of the injury. Animals that were treated with penicillinase or a CSF recovered function to an average BBB score of about 4 (n=11). Animals that were treated with Chondrointinase ABC 1 (n=7) recovered locomotor function to a BBB score of approximately 8. The chondroitinase ABC 1 treated group was significantly different from both other groups according to ANOVA and post-hoc Tukey analysis (p<0.01). See Figure 3 illustrating BBB scores of rats following contusive spinal cord injury with administration of aCSF, penicillinase or chondroitinase ABC 1.

These results demonstrate the potential use of a chondroitinase enzyme for the treatment of a contusive spinal cord injury. The spinal cord injury model and behavioral analysis system are well accepted in the field are believed to be the most relevant to most human spinal cord injuries. These results could not have been anticipated from the published results of chondrointinase *in vitro* or from the results of transaction injury models of the spinal cord.

### Example 5:

### Chondroitinase ABC_{TypeI} improves neurological function following contusion injury of the spinal cord in rodents

A study of chondroitinase ABC_{TypeI} treatment of a contusion injury in the rat was completed in the Acorda Animal Modeling Facility. Animals (n=38) were subjected to an established model of SCI (Gruner et al. 1996). Beginning immediately following the SCI, 19 animals were treated with Chondroitinase ABC 1 (Seikagaku; Cat number 100332, lot number E02201) intrathecally (i.t.) at 0.06 Units per rat per dose in artifical cerebrospinal fluid, every other day for two weeks. The other 19 animals were treated with enzymatic protein (Penicillinase - Sigma; Cat number P4524) in vehicle. Animals were evaluated by open-field behavioral testing for a period of 10 weeks.

The animals were induced and maintained in a state of surgical anesthesia with 1.5% isoflurane carried in medical grade air (95% oxygen, 5% CO₂) mixture. An initial dose of Cefazolin (50 mg/kg, s.c.) was given preoperative. During surgery, the animal were placed on a heating pad to help maintain body temperature, and the pulse rate, SpO2 and temperature of the animal were monitored. A laminectomy of the T9 and T10 spinal vertebrae was performed. A partial laminectomy at the T13 / L1 junction was made for the placement of an intrathecal catheter. An incision in the dura was made with a hypodermic needle. A 32 gauge catheter (ReCathCo., LLC, CS132G, lot number 20422) was inserted through the dural incision and fed rostally to lie immediately caudal to the T9 / T10 laminectomy. The catheter was secured to bone and muscle with cyanoacrylate glue and sutures. The blades of modified coverslip forceps (4 mm wide x 0.5 mm thick) were inserted into the spinal canal between the lateral aspects of the spinal cord and vertebrae at the rostral laminectomy. A SCI was induced by compressing the spinal cord between the blades of the forceps for a period of 15 seconds. The level of injury severity was induced by using forceps that compress the spinal cord to a distance of 0.9 mm (moderate injury). The dura over the injury remains intact. The forceps were removed, and the injury site flushed with saline. Overlaying muscle layers were sutured together and the skin wound was stapled closed. Post operatively, the animals were given an immediate 5 ml bolus of lactated Ringer's saline solution followed by a second administration of 5 mls lactated Ringer's saline solution after a few hours.

Immediately after surgery and then every other day for 2 weeks, animals were anesthetized with isoflurane and the experimental (Chondoitinase ABC I) or control agent (Penicillinase) as described below were injected into the intrathecal catheter. The volume injected was 3 microliters, followed by a 4 microliter wash of artificial cerebrospinal fluid.
Treatment Regimens:
1. Chase ABC 1 - Chondroitinase ABC 1 (Seikagaku), 0.06 U / dose, i.t. in 3 microliters aCSF.
2. Penicillinase - Penicillinase (Sigma), 3 microliters, i.t. at 228 micrograms per milliliter.

Behavioral Analysis: At 48 hours and then weekly after injury, open field locomotor activity was observed and scored according to the Basso, Beattie and Bresnahan (BBB) scoring system (Basso et al., 1995). This system is well accepted in the field for the assessment of recovery of locomotor function following spinal cord injury in the rat.

Results: Thirty-seven animals were enrolled: 19 per treatment group in the 0.9 mm forceps injury. Animal death was approximately equal among all treatment groups. Of the animals that died, more that 90% had urinary tract infections. One penicillinase treated 0.9 mm forceps injury animal was removed for scores that did not recover above a BBB of 3 or was extremely erratic. An additional 4 animals (2 penicillinase and 2 chondroitinase treated) were removed due to tremors and dyskinesias. Removal of these animals resulted in the number of animals in each 0.9 mm group to 12.

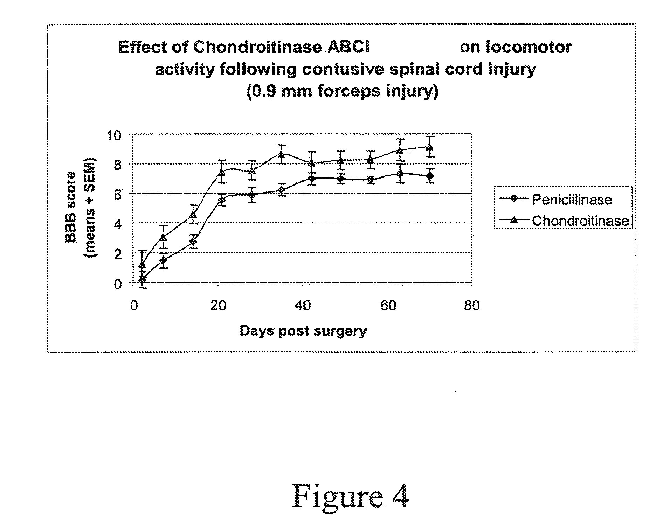

*0.9 mm forceps (moderate injury).* Animals that were treated with penicillinase control recovered function to a BBB score of 7.1 ± 0.36 (mean ± SEM) (n=12) at ten weeks post injury. Mean BBB scores of animals treated with Chondrointinase ABC I (n=12)were significantly higher at 10 weeks with an average score of 9.1 ± 0.64 (p<0.01). The scores of each group after reaching plateau at 4 weeks were also significantly different by ANOVA (p<0.001). See Figure 4 illustrating BBB scores of Chondroitinase and control animals following moderate contusive SCI.

Chondroitinase has been shown to improve function and promote regeneration in dorsal hemisection and a severe forceps compression model of SCI. The present study confirms the result of the forceps compression study as well as demonstrates that chondroitinase is effective at improving locomotor function at more moderate injury levels. The moderate injury study showed significant improvement in open-field locomotor activity with chondroitinases treatment.

### Example 6:

### Chondroitinase ABC_{TypeI} acute distribution and toxicity

Female Long Evans rats from Charles River Laboratories, weighing approximately 210 grams were housed in the Acorda Animal Care Facility for 5 days prior to infection to ensure health and weight stability. Rats were anesthetized with isoflurane and injected i.v. via tail veins with Acorda chondroitinase ABC I (ABCI-batch 3). Animals were injected with either 0, 0.2, 0.775 or 7.775 mg/kg with solutions containing 0, 0.2, 0.775 and 7.775 mg/ml, respective in Hank's balanced salt solution as shown in Table 1.

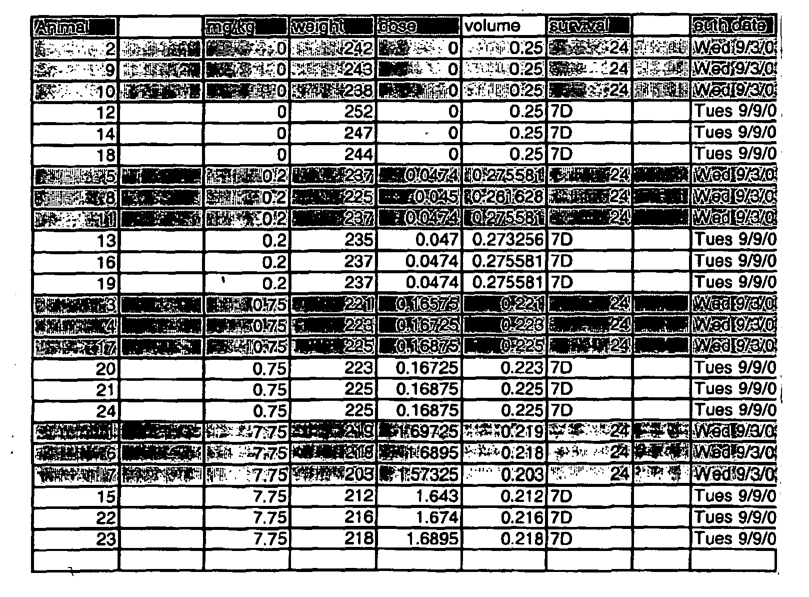

Animals were returned to their home cages and monitored for pain and distress. Weights were acquired daily.

Half of the animals were sacrificed at 24 hours after injection. Brain, spinal cord, heart, liver, kidney and blood were removed and rapidly frozen in isopentane cooled to -40°C for enzyme distribution assessment and histopathology. The remaining animals were observed for 7 days and then sacrificed and processed as with the 24 hour survival group.

Tissue was blocked and cryosectioned at 20 µm. Sections were washed briefly in 0.1 M phosphate buffered saline (PBS) and then fixed for 10 minutes in an ethanol, formalin acetic acid fixative (66, 4, 5 % by volume, respectively). Tissues were washed and blocked in a solution of 10% normal serum (containing 2% normal rat serum and 8% normal donkey serum) in 0.1 M PBS pH 7.4. Sections were incubated overnight in an anti-chondroitinase ABC I antibody (#8429). Tissue is assessed by immunohistochemistry and western blotting of tissue homogenates with an anti-chondroitinase and an antibody that recognized digested chondroitin sulfate proteoglycans.

Results: No overt reactions were observed during or immediately after injection. No swelling, inflammation, bruising or necrosis was noted at the injection site. No alterations in feeding, grooming or vocalizations were noted. Animals were assessed for motor behavior in an open pool. No abnormalities were noted by the animal care staff or behavioral specialists. Animals displayed to signs of joint tenderness or swelling.

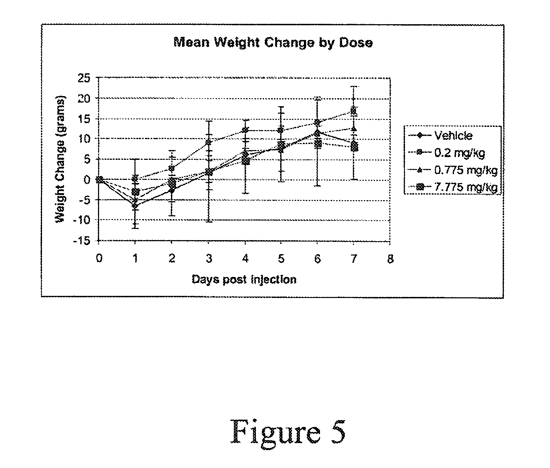

Animals were weighed every day prior to and following injections. The mean changes in body weight are illustrated in Figure 5. There were no significant differences in weight change between the treatment groups. All groups lost between 0 and 6.667 grams in the 24 hours following injection. The vehicle control group lost the most weight at 24 hours. All treatment groups gained weight on each successive day.

### Example 7:

### Chondroitinase ABC_{TypeI} single dose intrathecal toxicity

Intrathecal catheters were placed in 16 normal, un-injured female rats at about the T13/L1 vertebral junction for delivery of chondroitinase. Catheters were fed rostrally to rest at the T9/T10 level to simulate previous chondroitinase studies. Twenty-four hours after intrathecal catheter placement animals were dosed with 0, 0.06, 0.6 or 6.0 Units of Acorda chondroitinase ABCI (100 Units / milligram) in 20 microliters of artificial cerebrospinal fluid over a 20 minute period. These doses were chosen from 0, 1, 10 and 100 times the dose used in Examples 1, 4, and 5. Animals were observed for 24 hours or 7 days and their weights and temperatures were followed.

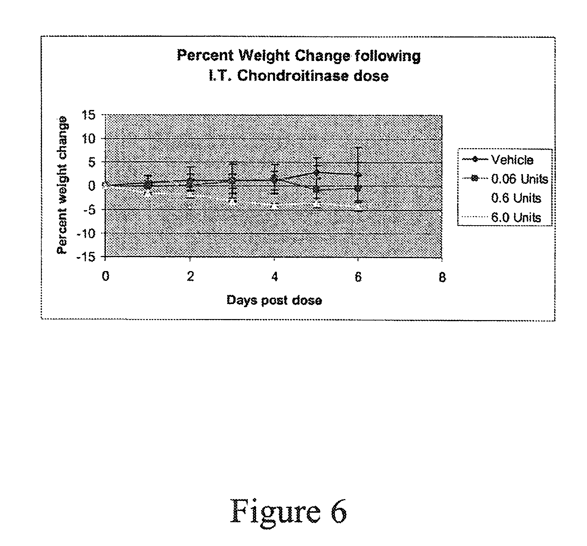

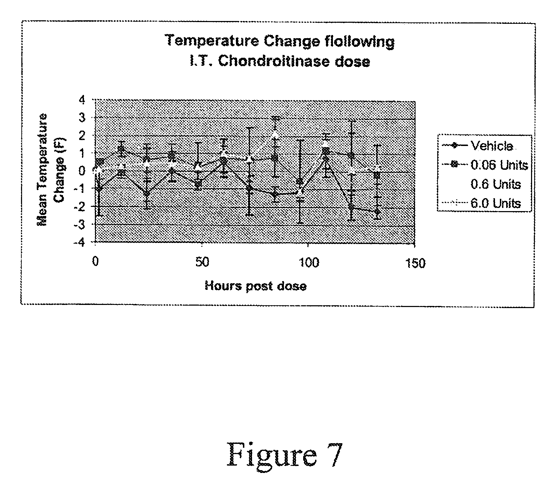

No overt reactions were seen in any rats. As illustrated in Figures 6 and 7, no significant differences in weight or temperature were noted between groups, respectively. Chondroitinase appears safe in single intrathecal (I.T.) doses at up to 100 times the doses used in previous studies.

### Example 8

### Chondroitinase ABC_{TypeI} repeat and dose escalation intrathecal toxicity study

Intrathecal catheters were placed in five adult female Long Evans rats. Rats were anesthetized, muscles cleared from the vertebrae and a small laminectomy performed at approximately the T13/L1 junction. Intrathecal catheters of 1.4 mm length were placed so that their tip ends at about the T9/T10 level. Rats were anesthetized with isoflurane and dosed with either vehicle (aCSF), repeat or escalating doses of chondroitinase ABCI. Repeat doses were 0.6 Units or 10 times the efficacious dose from previous studies. Escalating doses were: 0.6, 1.2, 2.4, 4.8, 9.6 and 19.2 Units. Rats were monitored for weight and temperature changes, overt toxicity and behavioral changes.

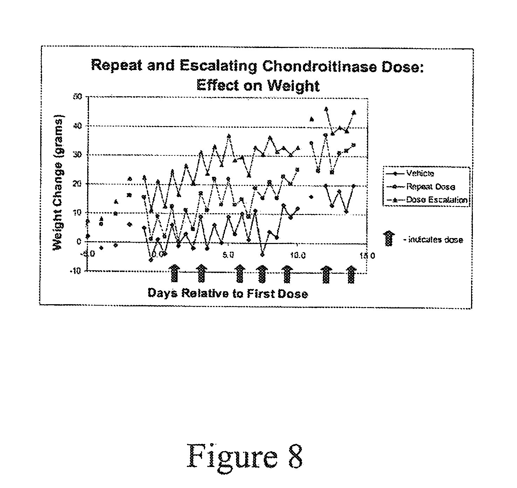

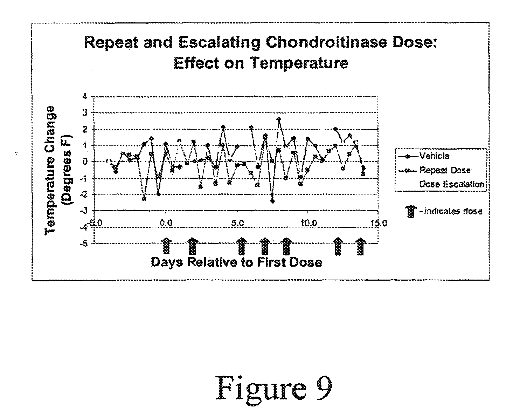

Rats treated with either repeat or escalating doses of chondroitinase ABCI gained slightly more weight than with vehicle. Adverse effects were evenly distributed between vehicle, repeat and escalating dose rats. Adverse events included lethargy and tail drop and usually occurred on the day of anesthesia and dosing. Scatter plot of weight change is shown in Figure 8 and temperature change is shown in Figure 9 during dosing regimen. In both figures, twice daily weights are plotted relative to the first dose. Timing of doses is indicated with the arrows.

### Example 9

### Chondroitinase improves neurological function in a chronic spinal cord contusion injury

Individuals that have injuries to the CNS recovery some degree of neurological function and then enter into a chronic phase of injury wherein limited improvement occurs. With the exception of the Examples herein, all of the studies to date with condroitinase have been conducted in animals immediately following transaction injuries of the spinal cord. In the example, chondroitinase ABC_{TypeI} chondroitinase ABC_{TypeII}, chondrointinase AC and chondrointinase B or mammalian enzymes with chondroitinase-like activity such as Hyal 1, Hyal 2. Hyal 3, and Hyal 4 are used to treat mammals in the chronic phase of injury following a confusion injury of the CNS. In this example, rats are subjected to a contusion injury of the spinal cord and allowed to recover for at least 6 weeks. At this stage the animals have all reached a plateau value for open field locomotion as assessed by the BBB scoring method described in Example 1. The animals are treated with chondroitinase ABC_{TypeI}, chondroitinase ABC_{TypeII}. chondroitinase AC and chondroitinase B or mammalian enzymes with chondroitinase-like activity such as Hyal 1, Hyal 2, Hyal 3, and Hyal 4.

### Example 10

### Cloning of chondroitinase AC from Flavobacterium heparinium:

*Flavobacterium heparinum* (ATCC) was grown in LB (Luria broth) at 25°C for 4 days. The bacteria were spun down by centrifugation and genomic DNA was isolated by DNeasy Tissue kit (Qiagen). PCR primers were synthesized with a *Nde*I restriction site at the 5' end and a *Bam*HI site at the 3' end having sequences 5' - CATATGCAGCAGACCGGTACTGCA-3' and 5'-GGATTCTCAGTGCTCTTTATTTCT-3' respectively to synthesize the mature protein. One microgram of the genomic DNA was used in a 50 µl PCR reaction containing 10mM of each dNTP (dATP, dTTP, dCTP and dGTP), 50 pmol each of forward and reverse primers, 1mM of MgSO₄, and 5 units of *Tfl* DNA polymerase (Promega). The hot start PCR reaction was initiated by denaturation at 95°C for 2 min followed by another cycle of denaturation at 94°C for 30 sec, annealing and extension at 58°C for 8 min for 30 cycles. The final extension was carried on at 72°C for 8 min before cooling at 4 C. The 2.0kb PCR product was ligated into pCR 2.1 vector (TOPO cloning kit, Invitrogen) and transformed into OneShot competent cells (Invitrogen). Plasmid DNA was isolated from a number of clones screened by digestion with EcoRl restriction enzyme and the positive clones were selected having the 2.0 kb insert. The integrity of the gene is confirmed by DNA sequencing and shows 100% identity with the published sequence (Genbank accession no. U27583). The nucleotide sequence of chondroitinase AC is SEQ ID No. 1.

### Example 11

### Cloning of chondroitinase B from Flavobacterium heparinum:

Chondroitinase B was cloned by a the same method as in Example 2 using primers with a *Nde*I restriction site at the 5' end and *Bam*HI site at the 3' end having sequences 5'-CATATGCAGGTTGTTGCTTCAAAT-3' and 5'-GGATCCTCAGTGCTCTTTATT-TCT-3' respectively to synthesize the mature protein. One microgram of the genomic DNA was used in a 50 µl PCR reaction containing 10mM of each dNTP (dATP, dTTP, dCTP and dGTP), 50 pmol each of forward and reverse primers, 1mM of MgSO₄. and 5 units of *Tfl* DNA polymerase (Promega). The hot start PCR reaction was initiated by denaturation at 95°C for 2 min followed a second cycle of denaturation at 94°C for 30 sec, annealing and extension at 58°C for 5 min for 30 cycles. The final extension was carried on at 72°C for 8 min before cooling at 4°C. The 1.6 kb PCR product ws ligated into pCR 2.1 vector (TOPO cloning kit, Invitrogen) and transformed into OneShot component cells (Invitrogen). Plasmid DNA was isolated from a number of clones screened by digestion with *Eco*RI restriction enzyme and the positive clones were selected having the 1.6 kb insert. The integrity of the gene is confirmed by DNA sequencing and shows 100% identify with the published sequence (accession no. U27584). The nucleotide sequence of chondroitinase B is SEQ ID No. 2.

### Example 12

### Cloning of chondroitinase ABC_{TypeI}

Genomic DNA was isolated from *Proteus vulgaris* using Dneasy Tissue kit (Qiagen). PCR primers were synthesized with an *Nde*I restriction site at the 5' end and a *Bam*HI site at the 3' end having sequences 5'- CAT ATG GCC ACC AGC AAT CCT GCA TTT G-3'(**F2**) and 5'-GGA TCC TCA AGG GAG TGG CGA GAG-3'(**R**) respectively, to synthesize the mature protein (2). The 3.0 kb PCR products were ligated into pCR 2.1 vector (TOPO cloning kit, Invitrogen) and transformed into DH5α competent cells (Invitrogen). Plasmid DNA was isolated from a number of clones screened by digestion with *Eco*RI restriction enzyme. The integrity of the gene was confirmed by repeated DNA sequencing and shows 99.7% and 99.5% identify at the nucleotide as well as amino acid level respectively, when compared with the published sequence. The nucleotide sequence for chondroitinase ABC1 is SEQ ID NO. 3. The sequence identity at the amino acid level is SEQ ID No. 4.

### Example 13

### Cloning of chondroitinase ABC_{TypeII}

Chondroitinase ABC II has been cloned from genomic DNA of *P.vulgaris.* Forward and reverse primers were designed having the sequences 5'TTA CCC ACT CTG TCT CAT GAA GCT TTC3' and 5'TTA CTT AAC TAA ATT AAT AAC AGT AGG3' respectively. A single PCT product of mol.wt. of 3.0 kb was isolated after 30 cycles of amplification of the *P.vulgaris* genomic DNA. The PCR product has been cloned in pCR2.1 vector and confirmed by restriction digest. The integrity of the gene was confirmed by DNA sequencing and shows 99% identify with the published sequence. The nucleotide sequence of chondroitinase ABCII is SEQ ID No.5.

The above discrepancies at the nucleotide level resulted in 98.3% identity at the amino acid level. The amino acid sequence of chondroitinase ABCII is SEQ ID No.6.

### Example 14

### Chondroitinase AC and B causes neurite outgrowth in vitro

Chase AC and B were tested for the ability to promote neurite outgrowth over an inhibitory CSPG substrate. A CSPG mixture (0.5 mg/ml; Chemicon) was spotted onto poly-I-lysine coated tissue culture plastic. Recombinant Chase AC or B was added to the plate in serum-free media at 0.5 or 0.1 mg/ml (respectively) for 3 hours at 37°C. Cortical neurons from embroyonic day 18 rat pups were plated onto the spots. Phase contact photomicrographs were acquired 48 hours after plating and the cells were assessed for neurite outgrowth. As seen in Figure 2, both cloned Chase AC and B enzymes promoted neurite outgrowth over the CSPG substrate, when compared with non-treated controls. Neurite promotion was similar to commercially available Chase enzymes at equal molar concentrations.

### Example 15

### A fusion protein of chrondoitinase and TAT cellular transduction peptide

The TAT fusion protein of chondroitinase suitable for use herein is described in commonly owned copending U.S. Patent Application Serial No. [not yet assigned] filed concurrently herewith and incorporated herein by reference. The TAT protein form the human immunodeficiency virus (HIV) contains a protein transduction domain (PTD) that is involved in the transduction of HIV into cells. The PTD contains an 11 amino acid domain (TAT peptide) that is responsible for the activity of the PTD. The TAT peptide can be linked to proteins and facilitate the transduction o the proteins into cells. The mechanism of transduction is independent of the molecular weight or chemical properties of the proteins that are linked to the TAT Peptide. Therefore, the TAT Peptide provides a method to deliver any protein into the cell cytoplasm. *In vivo* studies show that if a fusion protein consisting of the TAT Peptide linked to the 120 kd enzyme, beta-galactosidease (β-Gal), is injected into mice, than a robust delivery of β-Gal into a wide variety of cells is observed. In particular, β-Gal was observed in the CNS. Without the TAT Peptide β-Gal was not observed in the CNS. Thus, TAT Peptide fusion proteins cross the blood brain barrier and are also tranduced into cells. Transport across the blood brain barrier is normally restricted to certain hydrophobic small molecules and particular low molecular weight lipophilic peptides. Transport of proteins as large as β-Gal are not be possible without substantial disruption of the blood brain barrier, but the TAT Peptide facilitates transport while leaving the blood brain barrier intact.

The present invention is a chondroitinase enzyme functionally linked to the TAT Peptide (TAT-Chase). The first advantage is that TAT-Chase will cross the blood brain barrier and there TAT-Chase can be used systemically to treat spinal cord injury and related disorders of the CNS. The second advantage is that TAT-Chase will be transduced into cells and then degrade intracellular CSPGs stores. Therefore, TAT-Chase will degrade both extracellular and intracellular CSPGs.

### Example 16

### Encapsulated cells that release chondroitinase

The genes encoding chondroitinase AC, chondroitinase B, chondroitinase ABC_{TypeI}, chondroitinase ABC_{TypeII}, or other enzymes with chondroitinase-like activity such as Hyal1, Hyal2, Hyal3, and Hyal4 are transfected into an appropriate mammalian cell such as a CHO line. Cells that express catalytically active chondroitinase are cloned and expanded. The chondroitinase-expressing cell line is encapsulated using polymeric systems such as polyacryinitryl, polyvinyl chloride (PAN-PVC) that allow diffusion of nutrients and gasses, but prevents intrusion of host cells. When the capsule is implanted the chondroitinase-producing cell lines survive and continuously secrete chondroitinase, however the cells are not subjected to immune rejection because they are immunoisolated in the polymeric capsule. The advantage of this system is that instead of chondroitinase delivery through catheters or pumps, the chondroitinase is continually secreted. Furthermore, when treatment is no longer required the capsule can be retrieved to end the treatment interval. It is to be understood that the encapsulated cell based system herein is not only suitable for use in the treatment of contusion injuries to the CNS, but also are suitable for use in the treatment of other CNS injuries and disorders.

### Example 17

### Deletion mutants of chondroitinase that are biologically active

The deletion mutants of chondroitinase suitable for use herein is described in commonly owned co-pending U.S. Patent Application Serial No. [not yet assigned] filed concurrently herewith and incorporated herein by reference. Recombinantly produced chondroitinases AC and B have shown efficacy *in vitro* by overcoming the barrier of an inhibitory substrate border, such as aggrecan and resulting in neurite extension for rate cortical neurons. However, in order to facilitate effective transport of the above enzymes to the injury site, a systematic deletion analysis based on the available crystal structures was carried out in order to determine the minimally sized polypeptides capable of degrading CSPGs. The cleavage activity of all these mutants have been screened *in vitro* by zymographic assay using aggrecan as substrate. To date, a truncated polypeptide of chondroitinase AC (nΔ50-cΔ275) lacking 50 and 275 amino acids from the amino and carboxy termini respectively having a molecular weight of 38kDa compared to 75kDa of the full length protein was found to be the minimal size that retained activity as tested by zymography assay. However, the deletion mutant of chondroitinase B (nΔ120-cΔ120) lacking 120 amino acids from each of the amino and carboxy termini, having a molecular weight of 26kDa compared to 52kDa of the full length protein has shown to retain activity as well in zymography assay. The homogenously purified truncated enzymes will be characterized *in vitro* and will further be tested *in vivo* in parallel with the full length molecules to evaluate the potency as therapeutics for spinal cord injury. It is to be understood that deletion mutants herein are not only suitable for use in the treatment of contusion injuries to the CNS, but also are suitable for use in the treatment of other CNS injuries and disorders.

What has been described and illustrated herein are embodiments of the invention along with some of their variations. The terms, descriptions and figures used herein are set forth by way of illustration only and are not meant as limitations. Those skilled in the art will recognize that many variations are possible within the invention.

## Claims

1. A chondroitinase ABC enzyme for use in improving functional recovery following a contusion injury of the central nervous system, the enzyme being administered in a therapeutically effective amount.

2. The enzyme of claim 1, wherein the therapeutically effective amount of the chondroitinase ABC enzyme comprises an amount sufficient to degrade chondroitin sulfate proteoglycans.

3. The enzyme of claim 2, wherein the degradation of the chondroitin sulfate proteoglycans occurs at the site of the contusion injury.

4. The enzyme of claim 2, wherein the degradation of the chondroitin sulfate proteoglycans occurs outside the site of the contusion injury.

5. The enzyme of claim 1, wherein the therapeutically effective amount of the chondroitinase ABC enzyme comprises an amount sufficient to improve motor function, sensory function, autonomic function or a combination thereof.

6. The enzyme of any one of claims 1 to 5, wherein the chondroitinase is chondroitinase ABC_{TypeI}.

7. The enzyme of any one of claims 1 to 5, wherein the chondroitinase is chondroitinase ABC_{TypeII}.

8. The enzyme of any one of claims 1 to 7, wherein the contusion injury comprises a traumatic brain injury.

9. The enzyme of any one of claims 1 to 7, wherein the contusion injury comprises a spinal cord injury.

10. The enzyme of claim 9, wherein the spinal cord injury comprises a blunt force injury to the spinal cord.

11. The enzyme of either one of claims 9 and 10, wherein the gross morphology of the spinal cord is maintained.

12. The enzyme of claim 9, wherein the spinal cord injury comprises an injury resulting in a condition selected from the group consisting of monoplegia, diplegia, paraplegia, hemiplegia and quadriplegia.

13. The enzyme of any one of claims 1 to 7, wherein the contusion injury comprises tom or partially severed neurons.

14. The enzyme of any one of claims 1 to 7, wherein the contusion injury comprises crushed neurons.

15. The enzyme of any one of claims 1 to 7, wherein the contusion injury comprises compression of the central nervous system.

16. The enzyme of claim 15, wherein the compression is caused by a traumatic force to the spinal cord.

17. The enzyme of claim 15, wherein the compression is caused by a tumor, hemorrhage, infarction, infectious process, stenosis or ischemia.

## Patentansprüche

1. Ein Chondroitinase ABC-Enzym für den Einsatz zum Verbessern der funktionellen Erholung nach einer Quetschverletzung des zentralen Nervensystems, wobei das Enzym in einer therapeutisch wirksamen Menge verabreicht wird.

2. Enzym gemäß Anspruch 1, wobei die therapeutisch wirksame Menge des Chondroitinase ABC-Enzyms eine ausreichende Menge umfasst, um Chondroitinsulfat-Proteoglykane abzubauen.

3. Enzym gemäß Anspruch 2, wobei der Abbau der Chondroitinsulfat-Proteoglykane an der Stelle der Quetschverletzung erfolgt.

4. Enzym gemäß Anspruch 2, wobei der Abbau der Chondroitinsulfat-Proteoglykane außerhalb der Stelle der Quetschverletzung erfolgt.

5. Enzym gemäß Anspruch 1, wobei die therapeutisch wirksame Menge des Chondroitinase ABC-Enzyms eine ausreichende Menge umfasst, um Motorik, Sensorik, autonome Funktion oder eine Kombination davon zu verbessern.

6. Enzym gemäß einem der Ansprüche 1 bis 5, wobei die Chondroitinase Chondroitinase ABCTyp I ist.

7. Enzym gemäß einem der Ansprüche 1 bis 5, wobei die Chondroitinase Chondroitinase ABCTyp II ist.

8. Enzym gemäß einem der Ansprüche 1 bis 7, wobei die Quetschverletzung eine traumatische Hirnverletzung umfasst.

9. Enzym gemäß einem der Ansprüche 1 bis 7, wobei die Quetschverletzung eine Verletzung des Rückenmarks umfasst.

10. Enzym gemäß Anspruch 9, wobei die Verletzung des Rückenmarks eine durch stumpfe Gewalt herbeigeführte Verletzung des Rückenmarks umfasst.

11. Enzym gemäß einem der Ansprüche 9 und 10, wobei die makroskopische Morphologie des Rückenmarks erhalten wird.

12. Enzym gemäß Anspruch 9, wobei die Verletzung des Rückenmarks eine Verletzung umfasst, die zu einem Leiden führt, das aus der Gruppe, bestehend aus Monoplegie, Diplegie, Paraplegie, Hemiplegie und Tetraplegie, ausgewählt ist.

13. Enzym gemäß einem der Ansprüche 1 bis 7, wobei die Quetschverletzung gerissene oder teilweise durchtrennte Neuronen umfasst.

14. Enzym gemäß einem der Ansprüche 1 bis 7, wobei die Quetschverletzung zerquetschte Neuronen umfasst.

15. Enzym gemäß einem der Ansprüche 1 bis 7, wobei die Quetschverletzung Stauchung des zentralen Nervensystems umfasst.

16. Enzym gemäß Anspruch 15, wobei die Stauchung durch eine traumatische Kraft auf das Rückenmark verursacht wird.

17. Enzym gemäß Anspruch 15, wobei die Stauchung durch einen Tumor, Blutung, Herzinfarkt, infektiösen Prozess, Stenose oder Ischämie verursacht wird.

## Revendications

1. Une enzyme chondroïtinase ABC destinée à être utilisée dans l'amélioration de la récupération fonctionnelle à la suite d'une lésion par contusion du système nerveux central, l'enzyme étant administrée dans une quantité thérapeutiquement efficace.

2. L'enzyme de la revendication 1, dans laquelle la quantité thérapeutiquement efficace de l'enzyme chondroïtinase ABC comprend une quantité suffisante pour dégrader des protéoglycanes chondroïtine-sulfate.

3. L'enzyme de la revendication 2, dans laquelle la dégradation des protéoglycanes chondroïtine-sulfate se produit au site de la lésion par contusion.

4. L'enzyme de la revendication 2, dans laquelle la dégradation des protéoglycanes chondroïtine-sulfate se produit en dehors du site de la lésion par contusion.

5. L'enzyme de la revendication 1, dans laquelle la quantité thérapeutiquement efficace de l'enzyme chondroïtinase ABC comprend une quantité suffisante pour améliorer la fonction motrice, la fonction sensorielle, la fonction autonome ou une combinaison de celles-ci.

6. L'enzyme de n'importe laquelle des revendications 1 à 5, dans laquelle la chondroïtinase est la chondroïtinase ABC_{TypeI}.

7. L'enzyme de n'importe laquelle des revendications 1 à 5, dans laquelle la chondroïtinase est la chondroïtinase ABC_{TypeII}.

8. L'enzyme de n'importe laquelle des revendications 1 à 7, dans laquelle la lésion par contusion comprend une lésion cérébrale traumatique.

9. L'enzyme de n'importe laquelle des revendications 1 à 7, dans laquelle la lésion par contusion comprend une lésion de la moelle épinière.

10. L'enzyme de la revendication 9, dans laquelle la lésion de la moelle épinière comprend une lésion par force contondante sur la moelle épinière.

11. L'enzyme de l'une ou l'autre des revendications 9 et 10, dans laquelle la morphologie macroscopique de la moelle épinière est conservée.

12. L'enzyme de la revendication 9, dans laquelle la lésion de la moelle épinière comprend une lésion engendrant un état choisi dans le groupe consistant en une monoplégie, une diplégie, une paraplégie, une hémiplégie et une tétraplégie.

13. L'enzyme de n'importe laquelle des revendications 1 à 7, dans laquelle la lésion par contusion comprend des neurones déchirés ou partiellement sectionnés.

14. L'enzyme de n'importe laquelle des revendications 1 à 7, dans laquelle la lésion par contusion comprend des neurones écrasés.

15. L'enzyme de n'importe laquelle des revendications 1 à 7, dans laquelle la lésion par contusion comprend une compression du système nerveux central.

16. L'enzyme de la revendication 15, dans laquelle la compression est causée par une force traumatique sur la moelle épinière.

17. L'enzyme de la revendication 15, dans laquelle la compression est causée par une tumeur, une hémorragie, un infarctus, un processus infectieux, une sténose ou une ischémie.
